(19) **Europäisches Patentamt / European Patent Office / Office européen des brevets**

(11) **EP 4 682 259 A1**

(12) **EUROPEAN PATENT APPLICATION**
published in accordance with Art. 153(4) EPC

(43) Date of publication:
**21.01.2026 Bulletin 2026/04**

(21) Application number: **24770260.8**

(22) Date of filing: **01.02.2024**

(51) International Patent Classification (IPC):
*C12N 15/54* (2006.01)        *C12N 1/21* (2006.01)
*C12N 9/10* (2006.01)         *C12N 15/63* (2006.01)
*C12P 19/00* (2006.01)

(52) Cooperative Patent Classification (CPC):
**C12N 9/10; C12N 15/63; C12N 15/74; C12P 19/00**

(86) International application number:
**PCT/JP2024/003369**

(87) International publication number:
**WO 2024/190140 (19.09.2024 Gazette 2024/38)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **16.03.2023 JP 2023042144**

(71) Applicant: **FUJIFILM Corporation
Tokyo 106-8620 (JP)**

(72) Inventors:
• **TAKAHASHI, Motomasa
  Ashigarakami-gun, Kanagawa 258-8577 (JP)**
• **YAMAKAWA, Takayuki
  Ashigarakami-gun, Kanagawa 258-8577 (JP)**
• **TERADA, Daisuke
  Ashigarakami-gun, Kanagawa 258-8577 (JP)**
• **SONODA, Yuki
  Ashigarakami-gun, Kanagawa 258-8577 (JP)**
• **OTA, Tomoki
  Ashigarakami-gun, Kanagawa 258-8577 (JP)**

(74) Representative: **HGF
HGF Limited
4th Floor, 1 City Square
Leeds LS1 2AL (GB)**

(54) **MUTANT GLYCOSYLTRANSFERASE, METHOD FOR PRODUCING SAME, NUCLEIC ACID, VECTOR, HOST CELL, COMPOSITION, AND METHOD FOR PRODUCING ANTHRAQUINONE C-GLYCOSIDE**

(57)    Provided are a mutant glycosyltransferase having 80% or more sequence identity with an amino acid sequence set forth in SEQ ID NO: 1, in which the mutant glycosyltransferase is capable of forming an anthraquinone C-glycoside from an anthraquinone compound and a monosaccharide donor; a method for producing the same; a nucleic acid; a vector; a host cell; a composition; and a method for producing an anthraquinone C-glycoside.

**Description**

**BACKGROUND OF THE INVENTION**

1. Field of the Invention

The present disclosure a mutant glycosyltransferase, a method for producing the same, a nucleic acid, a vector, a host cell, a composition, and a method for producing an anthraquinone C-glycoside.

2. Description of the Related Art

Glycosyltransferase is a glycosyltransferase which catalyzes a reaction of transferring a sugar residue from a sugar donor such as a nucleotide sugar to a sugar receptor to form a glycosidic bond, and has been used in an industrial process for producing a glycoside using an enzymatic reaction.

Glycosides are classified into a C-glycoside, an O-glycoside, an N-glycoside, and the like depending on the type of an atom to which a sugar moiety and a non-sugar moiety are bonded. Among these, the C-glycoside (for example, an anthraquinone C-glycoside) in which the sugar moiety and the non-sugar moiety are bonded by a carbon-carbon bond is more likely to be stably present even in an acidic environment, as compared with the O-glycoside in which the sugar moiety and the non-sugar moiety are bonded by an oxygen-carbon bond. In particular, the anthraquinone C-glycoside (for example, a cochineal coloring agent) which is one of the C-glycosides is a useful compound which is widely used in dyes, foods, cosmetics, and the like, and an efficient production method thereof is expected.

US10100290B discloses a method for producing a cochineal coloring agent from kermesic acid or flavokermesic acid and glucose using a mutant glycosyltransferase derived from Coccoidea.

US2021/0261992A discloses a host cell which expresses a mutant glycosyltransferase for biosynthesis of carminic acid from polyketide building blocks.

In Dongsoo Yang, Woo Dae Jang, Sang Yup Lee, Journal of American Chemical Society, 2021, 143, 14, pp. 5364 to 5377, a novel Gentiana triflora-derived mutant glycosyltransferase having C-glycosylation activity, and a method for producing carminic acid from glucose using the same are disclosed.

**SUMMARY OF THE INVENTION**

However, in the conventional mutant glycosyltransferase, in a process in which a nucleic acid encoding a mutant glycosyltransferase is taken up into a host cell for expression, the amount of the mutant glycosyltransferase produced from the host cell per unit volume (hereinafter, also referred to as "titer") tends to be small due to reasons such as poor expression in the host cell, insolubilization of the protein, and inability to extract the protein. For example, in Dongsoo Yang, Woo Dae Jang, Sang Yup Lee, Journal of American Chemical Society, 2021, 143, 14, pp. 5364 to 5377, it is disclosed that, in a transformant in which Escherichia coli is used as the host cell, it is difficult to express a mutant glycosyltransferase (DcUGT2) in US10100290B and US2021/0261992A. In Dongsoo Yang, Woo Dae Jang, Sang Yup Lee, Journal of American Chemical Society, 2021, 143, 14, pp. 5364 to 5377, it is disclosed that a mutant glycosyltransferase (GtCGT) using Escherichia coli as the host cell can be expressed, but is insoluble in extraction from a transformant and has a low titer.

In view of the above-described circumstances, an object of the present disclosure is to provide a mutant glycosyltransferase having an excellent titer, a method for producing the same, a nucleic acid, a vector, a host cell, and a method for producing an anthraquinone C-glycoside.

The present disclosure includes the following aspects.

<1> A mutant glycosyltransferase having 80% or more sequence identity with an amino acid sequence set forth in SEQ ID NO: 1,
in which the mutant glycosyltransferase is capable of forming an anthraquinone C-glycoside from an anthraquinone compound and a monosaccharide donor.
<2> The mutant glycosyltransferase according to <1>,

in which at least one requirement selected from the group consisting of the following (1-A) to (6-A) is satisfied,
(1-A): an amino acid residue corresponding to a 17th position from an N-terminus of SEQ ID NO: 1 is a basic amino acid residue, a polar uncharged amino acid residue, or an aliphatic amino acid residue,
(2-A): an amino acid residue corresponding to a 20th position from the N-terminus of SEQ ID NO: 1 is a polar uncharged amino acid residue or an aliphatic amino acid residue,
(3-A): an amino acid residue corresponding to a 318th position from the N-terminus of SEQ ID NO: 1 is a basic amino acid residue, a polar uncharged amino acid residue, an aliphatic amino acid residue, or an aromatic amino acid residue,

(4-A): an amino acid residue corresponding to a 320th position from the N-terminus of SEQ ID NO: 1 is a basic amino acid residue, an acidic amino acid residue, a polar uncharged amino acid residue, an aliphatic amino acid residue, or an aromatic amino acid residue,

(5-A): an amino acid residue corresponding to a 382nd position from the N-terminus of SEQ ID NO: 1 is an aromatic amino acid residue,

(6-A): an amino acid residue corresponding to a 46th position from the N-terminus of SEQ ID NO: 1 is a polar uncharged amino acid residue, an aliphatic amino acid residue, or an aromatic amino acid residue.

<3> The mutant glycosyltransferase according to <2>,

in which at least one requirement selected from the group consisting of the following (1-B) to (6-B) is satisfied,

(1-B): the amino acid residue corresponding to the 17th position from the N-terminus of SEQ ID NO: 1 is a histidine residue (H), a serine residue (S), or a glycine residue (G),

(2-B): the amino acid residue corresponding to the 20th position from the N-terminus of SEQ ID NO: 1 is a cysteine residue (C), an asparagine residue (N), an isoleucine residue (I), or a valine residue (V),

(3-B): the amino acid residue corresponding to the 318th position from the N-terminus of SEQ ID NO: 1 is a histidine residue (H), an arginine residue (R), a cysteine residue (C), an asparagine residue (N), a serine residue (S), an isoleucine residue (I), a leucine residue (L), a valine residue (V), a phenylalanine residue (F), or a tyrosine residue (Y),

(4-B): the amino acid residue corresponding to the 320th position from the N-terminus of SEQ ID NO: 1 is a histidine residue (H), an arginine residue (R), an aspartic acid residue (D), a cysteine residue (C), a serine residue (S), a glycine residue (G), an isoleucine residue (I), a valine residue (V), a phenylalanine residue (F), or a tyrosine residue (Y),

(5-B): the amino acid residue corresponding to the 382nd position from the N-terminus of SEQ ID NO: 1 is a tyrosine residue (Y),

(6-B): the amino acid residue corresponding to the 46th position from the N-terminus of SEQ ID NO: 1 is a cysteine residue (C), a serine residue (S), a glycine residue (G), a valine residue (V), a phenylalanine residue (F), or a tyrosine residue (Y).

<4> The mutant glycosyltransferase according to <3>,

in which, among the requirements (1-B) to (6-B),
a combination of only (1-B) and (2-B),
a combination of only (3-B) and (4-B),
a combination of only (1-B), (2-B), and (5-B),
a combination of only (1-B), (2-B), (5-B), and (6-B),
a combination of only (1-B), (2-B), (3-B), (5-B), and (6-B),
a combination of only (1-B), (2-B), (4-B), (5-B), and (6-B), or
a combination of (1-B), (2-B), (3-B), (4-B), (5-B), and (6-B) is satisfied.

<5> The mutant glycosyltransferase according to any one of <1> to <4>,
in which the mutant glycosyltransferase has one amino acid sequence selected from the group consisting of SEQ ID NO: 1 to SEQ ID NO: 71.

<6> A nucleic acid encoding the mutant glycosyltransferase according to any one of <1> to <5>.

<7> A vector comprising:
the nucleic acid according to <6>.

<8> The vector according to <7>,
in which the vector is an expression vector.

<9> A host cell comprising:
the expression vector according to <8>.

<10> The host cell according to <9>,
in which the host cell is Escherichia coli.

<11> A method for producing a mutant glycosyltransferase, comprising:

culturing the host cell according to <9> in a culture medium; and
recovering the mutant glycosyltransferase from at least one of the cultured host cell or the culture medium.

<12> A composition comprising:
the mutant glycosyltransferase according to any one of <1> to <5>.
<13> A method for producing an anthraquinone C-glycoside, comprising:
a step of bringing an anthraquinone compound and a monosaccharide donor into contact with the mutant glycosyltransferase according to any one of <1> to <5> to form an anthraquinone C-glycoside.

According to the present disclosure, it is possible to provide a mutant glycosyltransferase having an excellent titer, a method for producing the same, a nucleic acid, a vector, a host cell, and a method for producing an anthraquinone C-glycoside.

## DESCRIPTION OF THE PREFERRED EMBODIMENTS

Hereinafter, embodiments according to the present disclosure will be described. Descriptions and Examples are only illustrative of the embodiments, and do not limit the scope of the embodiments.

In a numerical range described in a stepwise manner in the present disclosure, an upper limit or a lower limit described in one numerical range may be replaced with an upper limit or a lower limit in another numerical range described in a stepwise manner. In addition, in a numerical range described in the present disclosure, an upper limit or a lower limit described in the numerical range may be replaced with a value described in an example.

In the present disclosure, each component may contain a plurality of types of corresponding substances.

In the present disclosure, the meaning of the term "step" includes not only an independent step but also a step whose intended purpose is achieved even in a case where the step is not clearly distinguished from other steps.

In the present disclosure, the numerical ranges shown using "to" indicate ranges including the numerical values described before and after "to" as the minimum value and the maximum value.

Hereinafter, glycosyltransferase activity of obtaining an anthraquinone C-glycoside from an anthraquinone compound and a monosaccharide donor is also simply referred to as "C-glycosylation activity".

In the present disclosure, the description of nucleotide sequence may be made by focusing on a sequence on one strand even in a case where a nucleic acid chain holding the nucleotide sequence forms a double strand; but in the other strand of the double strand, the description of such a sequence should be applied by being replaced with the complementary sequence.

In the present disclosure, an amino acid residue may be represented by one alphabetic character. For example, a tryptophan residue may be represented by "W".

In the present disclosure, unless otherwise specified, even in a case where an element is expressed in a singular form, a plurality of elements are not excluded unless a technical contradiction occurs.

<Mutant glycosyltransferase>

The mutant glycosyltransferase according to the present disclosure has 80% or more sequence identity with an amino acid sequence set forth in SEQ ID NO: 1, in which the mutant glycosyltransferase is capable of forming an anthraquinone C-glycoside from an anthraquinone compound and a monosaccharide donor.

The mutant glycosyltransferase according to the present disclosure has 80% or more of sequence identity with the amino acid sequence set forth in SEQ ID NO: 1, and thus has an excellent titer.

The mutant glycosyltransferase according to the present disclosure has 80% or more sequence identity with the amino acid sequence set forth in SEQ ID NO: 1; and from the viewpoint of being more excellent in the titer and the C-glycosylation activity, it preferably has 84% or more sequence identity, more preferably has 90% or more sequence identity, still more preferably has 95% or more sequence identity, and particularly preferably has 97% or more sequence identity.

The mutant glycosyltransferase according to the present disclosure may have 99.9% or less sequence identity with an amino acid sequence set forth in SEQ ID NO: 75 (that is, an amino acid sequence of a wild-type glycosyltransferase derived from Punica granatum).

In the present disclosure, the mutant glycosyltransferase refers to a glycosyltransferase having one or a plurality of mutations with respect to the wild-type glycosyltransferase derived from Punica granatum.

The amino acid sequence set forth in SEQ ID NO: 1 is a sequence in which a histidine residue (H), which is an amino acid residue corresponding to the 20th position from the N-terminus, is changed to a valine residue (V), with respect to the amino acid sequence of the wild-type glycosyltransferase derived from Punica granatum, set forth in SEQ ID NO: 75.

In the amino acid sequence set forth in SEQ ID NO: 1, it is considered that the mutation of substituting the histidine residue (H), which is an amino acid residue corresponding to the 20th position from the N-terminus, with a valine residue (V) with respect to the amino acid sequence of the wild-type glycosyltransferase derived from Punica granatum set forth in SEQ ID NO: 75 results in an excellent titer.

(Mutation position)

In the mutant glycosyltransferase according to the present disclosure, from the viewpoint of more excellent titer and C-glycosylation activity, it is preferable to satisfy at least one requirement selected from the group consisting of the following (1-A) to (6-A), it is more preferable to satisfy two or more and six or less of the requirements, and it is still more preferable to satisfy three or more and six or less of the requirements:

(1-A): an amino acid residue corresponding to a 17th position from the N-terminus of SEQ ID NO: 1 is a basic amino acid residue, a polar uncharged amino acid residue, or an aliphatic amino acid residue;

(2-A): an amino acid residue corresponding to a 20th position from the N-terminus of SEQ ID NO: 1 is a polar uncharged amino acid residue or an aliphatic amino acid residue;

(3-A): an amino acid residue corresponding to a 318th position from the N-terminus of SEQ ID NO: 1 is a basic amino acid residue, a polar uncharged amino acid residue, an aliphatic amino acid residue, or an aromatic amino acid residue;

(4-A): an amino acid residue corresponding to a 320th position from the N-terminus of SEQ ID NO: 1 is a basic amino acid residue, an acidic amino acid residue, a polar uncharged amino acid residue, an aliphatic amino acid residue, or an aromatic amino acid residue;

(5-A): an amino acid residue corresponding to a 382nd position from the N-terminus of SEQ ID NO: 1 is an aromatic amino acid residue;

(6-A): an amino acid residue corresponding to a 46th position from the N-terminus of SEQ ID NO: 1 is a polar uncharged amino acid residue, an aliphatic amino acid residue, or an aromatic amino acid residue.

In the mutant glycosyltransferase according to the present disclosure, from the viewpoint of more excellent titer and C-glycosylation activity, it is preferable to satisfy at least one requirement selected from the group consisting of the following (1-A2) to (6-A2), it is more preferable to satisfy two or more and six or less of the requirements, and it is still more preferable to satisfy three or more and six or less of the requirements:

(1-A2): the amino acid residue corresponding to the 17th position from the N-terminus of SEQ ID NO: 1 is a polar uncharged amino acid residue;

(2-A2): the amino acid residue corresponding to the 20th position from the N-terminus of SEQ ID NO: 1 is a polar uncharged amino acid residue;

(3-A2): the amino acid residue corresponding to the 318th position from the N-terminus of SEQ ID NO: 1 is a basic amino acid residue or an aromatic amino acid residue;

(4-A2): the amino acid residue corresponding to the 320th position from the N-terminus of SEQ ID NO: 1 is a basic amino acid residue or an aliphatic amino acid residue;

(5-A2): the amino acid residue corresponding to the 382nd position from the N-terminus of SEQ ID NO: 1 is an aromatic amino acid residue;

(6-A2): the amino acid residue corresponding to the 46th position from the N-terminus of SEQ ID NO: 1 is an aromatic amino acid residue.

The basic amino acid residue refers to an amino acid residue composed of a basic amino acid.

The acidic amino acid residue refers to an amino acid residue composed of an acidic amino acid.

The aromatic amino acid residue refers to an amino acid residue composed of an aromatic amino acid. The aromatic amino acid refers to an amino acid having an aromatic group (for example, a phenyl group or the like), which is neither a basic amino acid nor an acidic amino acid.

The polar uncharged amino acid residue refers to an amino acid residue composed of a polar uncharged amino acid. The polar uncharged amino acid refers to an amino acid having a polar group (for example, a thiol group or the like) and not having a charge as a whole molecule in water (25°C), which is neither an acidic amino acid, a basic amino acid, nor an aromatic amino acid.

The aliphatic amino acid residue refers to an amino acid residue composed of an aliphatic amino acid. The aliphatic amino acid refers to an aliphatic amino acid which is neither a basic amino acid, an acidic amino acid, a polar uncharged amino acid, nor an aromatic amino acid.

The amino acid residue at the 17th position from the N-terminus of SEQ ID NO: 1 refers to a glycine residue (G).

The amino acid residue at the 20th position from the N-terminus of SEQ ID NO: 1 refers to a valine residue (V).

The amino acid residue at position 318 from the N-terminus of SEQ ID NO: 1 refers to a serine residue (S).

The amino acid residue at position 320 from the N-terminus of SEQ ID NO: 1 refers to a tyrosine residue (Y).

The amino acid residue at position 382 from the N-terminus of SEQ ID NO: 1 refers to a tryptophan residue (W).

The amino acid residue at the 46th position from the N-terminus of SEQ ID NO: 1 refers to an isoleucine residue (I).

Specific examples of the basic amino acid residue (referred to as "Basic" in the table), the acidic amino acid residue (referred to as "Acidic" in the table), the polar uncharged amino acid residue (referred to as "Polar uncharged" in the table), the aliphatic amino acid residue (referred to as "Aliphatic" in the table), and the aromatic amino acid residue (referred to as "Aromatic" in the table) described above are shown in the following table; but the amino acids are not limited thereto, and modified amino acid residues, unusual amino acid residues, and the like are also included.

[Table 1]

| Amino acid residue properties | | Amino acid residue | |
|---|---|---|---|
| | | One letter abbreviation | Name |
| Hydrophilic | Basic | H | Histidine |
| | | K | Lysine |
| | | R | Arginine |
| | Acidic | D | Aspartic acid |
| | | E | Glutamic acid |
| | Polar uncharged | C | Cysteine |
| | | N | Asparagine |
| | | Q | Glutamine |
| | | S | Serine |
| | | T | Threonine |
| Hydrophobic | Aliphatic | G | Glycine |
| | | A | Alanine |
| | | I | Isoleucine |
| | | V | Valine |
| | | L | Leucine |
| | | M | Methionine |
| | | P | Proline |
| | Aromatic | F | Phenylalanine |
| | | Y | Tyrosine |
| | | W | Tryptophan |

In the mutant glycosyltransferase according to the present disclosure, from the viewpoint of more excellent titer and C-glycosylation activity, it is preferable to satisfy at least one requirement selected from the group consisting of the following (1-B) to (6-B), it is more preferable to satisfy two or more and six or less of the requirements, and it is still more preferable to satisfy three or more and six or less of the requirements:

(1-B): the amino acid residue corresponding to the 17th position from the N-terminus of SEQ ID NO: 1 is a histidine residue (H), a serine residue (S), or a glycine residue (G);
(2-B): the amino acid residue corresponding to the 20th position from the N-terminus of SEQ ID NO: 1 is a cysteine residue (C), an asparagine residue (N), an isoleucine residue (I), or a valine residue (V);
(3-B): the amino acid residue corresponding to the 318th position from the N-terminus of SEQ ID NO: 1 is a histidine residue (H), an arginine residue (R), a cysteine residue (C), an asparagine residue (N), a serine residue (S), an isoleucine residue (I), a leucine residue (L), a valine residue (V), a phenylalanine residue (F), or a tyrosine residue (Y);
(4-B): the amino acid residue corresponding to the 320th position from the N-terminus of SEQ ID NO: 1 is a histidine residue (H), an arginine residue (R), an aspartic acid residue (D), a cysteine residue (C), a serine residue (S), a glycine residue (G), an isoleucine residue (I), a valine residue (V), a phenylalanine residue (F), or a tyrosine residue (Y);
(5-B): the amino acid residue corresponding to the 382nd position from the N-terminus of SEQ ID NO: 1 is a tyrosine residue (Y);

(6-B): the amino acid residue corresponding to the 46th position from the N-terminus of SEQ ID NO: 1 is a cysteine residue (C), a serine residue (S), a glycine residue (G), a valine residue (V), a phenylalanine residue (F), or a tyrosine residue (Y).

In the mutant glycosyltransferase according to the present disclosure, from the viewpoint of more excellent titer and C-glycosylation activity, it is preferable to satisfy at least one requirement selected from the group consisting of the following (1-B2) to (6-B2), it is more preferable to satisfy two or more and six or less of the requirements, and it is still more preferable to satisfy three or more and six or less of the requirements:

(1-B2): the amino acid residue corresponding to the 17th position from the N-terminus of SEQ ID NO: 1 is a serine residue (S);

(2-B2): the amino acid residue corresponding to the 20th position from the N-terminus of SEQ ID NO: 1 is an asparagine residue (N);

(3-B2): the amino acid residue corresponding to the 318th position from the N-terminus of SEQ ID NO: 1 is an arginine residue (R) or a phenylalanine residue (F);

(4-B2): the amino acid residue corresponding to the 320th position from the N-terminus of SEQ ID NO: 1 is an arginine residue (R) or a valine residue (V);

(5-B2): the amino acid residue corresponding to the 382nd position from the N-terminus of SEQ ID NO: 1 is a tyrosine residue (Y);

(6-B2): the amino acid residue corresponding to the 46th position from the N-terminus of SEQ ID NO: 1 is a phenylalanine residue (F).

In the mutant glycosyltransferase according to the present disclosure, from the viewpoint of more excellent titer and C-glycosylation activity, among the requirements (1-B) to (5-B), it is preferable to satisfy a combination of only (1-B) and (2-B), a combination of only (3-B) and (4-B), a combination of only (1-B), (2-B), and (5-B), a combination of only (1-B), (2-B), (5-B), and (6-B), a combination of only (1-B), (2-B), (3-B), (5-B), and (6-B), a combination of only (1-B), (2-B), (4-B), (5-B), and (6-B), or a combination of (1-B), (2-B), (3-B), (4-B), (5-B), and (6-B); it is more preferable to satisfy a combination of (1-B) and (2-B), a combination of only (1-B), (2-B), and (5-B), a combination of (1-B), (2-B), (5-B), and (6-B), or a combination of (1-B), (2-B), (3-B), (4-B), (5-B), and (6-B); and it is still more preferable to satisfy a combination of only (1-B), (2-B), and (5-B), a combination of (1-B), (2-B), (5-B), and (6-B), or a combination of (1-B), (2-B), (3-B), (4-B), (5-B), and (6-B).

In the above description, for example, the "satisfy a combination of only (1-B) and (2-B)" means that, among (1-B) to (6-B) only (1-B) and (2-B) are satisfied and (3-B), (4-B), (5-B), and (6-B) are not satisfied. In this case, conditions other than (1-B) to (6-B) are not particularly limited. For example, amino acid residues other than the amino acid residues at the 17th, 20th, 46th, 318th, 320th, and 382nd positions from the N-terminus of SEQ ID NO: 1 are not particularly limited.

In the mutant glycosyltransferase according to the present disclosure, as long as the sequence identity with the amino acid sequence set forth in SEQ ID NO: 1 is 80% or less, the mutant glycosyltransferase may not satisfy all of the requirements represented by (1-A) to (6-A) and (1-B) to (6-B) described above.

The mutant glycosyltransferase according to the present disclosure may further include amino acid residue substitutions other than the requirements represented by (1-A) to (6-A) and (1-B) to (6-B) described above within a range in which a titer can be obtained.

In the mutant glycosyltransferase, the total number of requirements selected from the group consisting of (1-A) to (6-A) and (1-B) to (6-B) described above is not particularly limited as long as it is 1 or more, and it may be, for example, any of 1 to 10, 1 to 8, 1 to 7, 1 to 6, 1 to 5, 1 to 4, 1 to 3, 1 and 2, or 1.

From the viewpoint of more excellent titer and C-glycosylation activity, the mutant glycosyltransferase preferably has one amino acid sequence selected from the group consisting of SEQ ID NO: 1 to SEQ ID NO: 71, and more preferably has one amino acid sequence of SEQ ID NO: 59, SEQ ID NO: 70, or SEQ ID NO: 71.

The titer of the mutant glycosyltransferase according to the present disclosure is preferably 1 (mg/L) or more, more preferably 3 (mg/L) or more, and still more preferably 10 (mg/L) or more.

The C-glycosylation activity of the mutant glycosyltransferase according to the present disclosure is preferably 1 (pkat/mg) or more, more preferably 10 (pkat/mg) or more, and still more preferably 100 (pkat/mg) or more.

The C-glycosylation activity of the mutant glycosyltransferase according to the present disclosure, which can be extracted from a culture solution, is preferably 10 (pkat/L) or more, more preferably 100 (pkat/L) or more, and still more preferably 1,000 (pkat/L) or more.

Each of the values of the C-glycosylation activity, the C-glycosylation activity, and the C-glycosylation activity which can be extracted from a culture solution is determined as follows. Specifically, each of the values is determined by methods described in Examples later.

(Titer)

For an enzyme solution to be tested, an enzyme amount (that is, an amount of the mutant glycosyltransferase) is quantified from a concentration of the enzyme solution based on the absorbance at 280 nm measured with a spectrophotometer. Subsequently, the enzyme diluted solution is developed by SDS-PAGE, the gel is stained with a Coomassie Brilliant Blue (CBB) staining solution, and then the purity of a band containing a target protein (that is, a band corresponding to the mutant glycosyltransferase) is quantified by image analysis. From the acquired enzyme amount and the culture solution amount, the titer is calculated based on the following expression.

Enzyme amount (mg) = Enzyme solution concentration (mg/L) $\times$ Enzyme solution amount (L) $\times$ (Purity (%)/100)

$$\text{Titer (mg/L)} = \text{Enzyme amount (mg)/Culture solution amount (L)}$$

(C-glycosylation activity)

A DMSO solution of 0.5 mM of an anthraquinone compound, an aqueous solution of 5 mM of a monosaccharide donor, and 20 $\mu$g of an enzyme to be tested are stirred and reacted at 25°C for a sufficient time in 100 $\mu$L of a buffer (mixed solution of 50 mM of HEPES (pH: 7.5), 1 mg/mL of lysozyme, 0.5 mg/mL of polymyxin B, 13 mM of $MgCl_2$, and 50 mM of KCl). Thereafter, 100 $\mu$L of methanol is added to the reaction system to terminate the reaction, and centrifugation (4°C, 1,800 G, 30 min) is carried out to obtain a supernatant.

The supernatant is subjected to high performance liquid chromatography (HPLC) measurement, and the amount of the target product, anthraquinone C-glycoside, is quantitatively analyzed. The HPLC analysis conditions are as follows. In addition, as the device and the column, the same device and column as described below may be used.

-HPLC analysis conditions-

Device: Nexera X2 (manufactured by Shimadzu Corporation)
Column: Waters ACQUITY UPLC BEH C18, 1.7 $\mu$m, 2.0 $\times$ 50 mm
Mobile phase: A: water (0.1% formic acid), B: methanol (0.1% formic acid)
Time (min)/%B: 0/10, 0.4/10, 3.0/80, 4.0/100, 5.0/100, 5.1/10
Injection volume: 2 $\mu$L
Column temperature: 40°C
Flow rate: 0.4 mL/min

The C-glycosylation activity is calculated from the amount of the anthraquinone C-glycoside produced, the reaction time, and the amount of enzyme used by HPLC according to the following expression.

C-glycosylation activity (pkat/mg) = Amount of produced C-glycoside (pmol)/Reaction time (s)/Amount of enzyme used (mg)

(C-glycosylation activity which can be extracted from culture solution)

From the C-glycosylation activity and the titer obtained by the above-described evaluations, the enzyme activity which can be extracted from the culture solution is calculated based on the following expression.

Enzyme activity (pkat/L) which can be extracted from culture solution = Anthraquinone C-glycosylation activity (pkat/mg) $\times$ Expression efficiency (mg/L)

The amino acid residue substitution with respect to the amino acid sequence set forth in SEQ ID NO: 1, which is included in the mutant glycosyltransferase according to the present disclosure, is exemplified below, but the present disclosure is not limited thereto. In the tables, for example, "G17H" described in the item of [Effective mutation point] indicates that the amino acid residue corresponding to the 17th position from the N-terminus of SEQ ID NO: 1 is replaced with a histidine residue (H) from a glycine residue (G).

[Table 2]

|   | Effective mutation point |
|---|---|
| 2 | H19V |
| 3 | G17H |
| 4 | G17S |
| 5 | V20C |
| 6 | G17H, V20C |
| 7 | G17H, V20N |
| 8 | G17H, V20I |
| 9 | G17S, V20C |
| 10 | G17S, V20N |
| 11 | G17S, V20I |
| 12 | S318R |
| 13 | Y320H |
| 14 | Y320R |
| 15 | Y320V |
| 16 | S318H, Y320R |
| 17 | S318H, Y320V |
| 18 | S318R, Y320H |
| 19 | S318R, Y320R |
| 20 | S318R, Y320C |

[Table 3]

|   | Effective mutation point |
|---|---|
| 21 | S318R, Y320S |
| 22 | S318R, Y320I |
| 23 | S318R, Y320V |
| 24 | S318N, Y320V |
| 25 | S318G, Y320R |
| 26 | S318I, Y320R |
| 27 | S318I, Y320I |
| 28 | S318I, Y320V |
| 29 | S318L, Y320R |
| 30 | S318V, Y320R |
| 31 | S318V, Y320I |
| 32 | S318V, Y320L |
| 33 | S318V, Y320V |
| 34 | S318F, Y320R |
| 35 | S318F, Y320I |
| 36 | S318F, Y320V |
| 37 | S318Y, Y320I |

(continued)

|    | Effective mutation point |
|----|---------------------------|
| 38 | S318Y, Y320V |
| 39 | W382Y |
| 40 | G17S, V20N, W382Y |

[Table 4]

|    | Effective mutation point |
|----|---------------------------|
| 41 | G17S, V20N, W382Y, I46C |
| 42 | G17S, V20N, W382Y, I46S |
| 43 | G17S, V20N, W382Y, I46G |
| 44 | G17S, V20N, W382Y, 146V |
| 45 | G17S, V20N, W382Y, I46F |
| 46 | G17S, V20N, W382Y, I46Y |

[Table 5]

|    | Effective mutation point |
|----|---------------------------|
| 47 | G17S, V20N, S318R, W382Y, I46F |
| 48 | G17S, V20N, S318V, W382Y, I46F |
| 49 | G17S, V20N, Y320R, W382Y, I46F |
| 50 | G17S, V20N, Y320, W382Y, I46F |
| 51 | G17S, V20N, S318H, Y320R, W382Y, I46F |
| 52 | G17S, V20N, S318H, Y320V, W382Y, I46F |
| 53 | G17S, V20N, S318R, Y320H, W382Y, I46F |
| 54 | G17S, V20N, S318R, Y320R, W382Y, I46F |
| 55 | G17S, V20N, S318R, Y320D, W382Y, I46F |
| 56 | G17S, V20N, S318R, Y320C, W382Y, I46F |
| 57 | G17S, V20N, S318R, Y320G, W382Y, I46F |
| 58 | G17S, V20N, S318R, Y320I, W382Y, I46F |
| 59 | G17S, V20N, S318R, Y320V, W382Y, I46F |
| 60 | G17S, V20N, S318R, Y320F, W382Y, I46F |
| 61 | G17S, V20N, S318C, Y320R, W382Y, I46F |
| 62 | G17S, V20N, S318G, Y320R, W382Y, I46F |
| 63 | G17S, V20N, S318I, Y320R, W382Y, I46F |
| 64 | G17S, V20N, S318I, Y320V, W382Y, I46F |
| 65 | G17S, V20N, S318L, Y320V, W382Y, I46F |
| 66 | G17S, V20N, S318V, Y320R, W382Y, I46F |
| 67 | G17S, V20N, S318V, Y320L, W382Y, I46F |
| 68 | G17S, V20N, S318V, Y320V, W382Y, I46F |
| 69 | G17S, V20N, S318V, Y320F, W382Y, I46F |
| 70 | G17S, V20N, S318F, Y320R, W382Y, 146F |

(continued)

|  | Effective mutation point |
| --- | --- |
| 71 | G17S, V20N, S318F, Y320V, W382Y, I46F |

Alignment between sequences can be performed by pairwise alignment using BLAST (registered trademark) which is a sequence homology search program. The parameter is a default parameter. The sequence identity is calculated based on the full length of glycosyltransferase.

In a case where the amino acid sequence set forth in SEQ ID NO: 1 is aligned with the amino acid sequence to be compared, a distance from the defined N-terminus may vary depending on the amino acid sequence to be compared, even in a case where the corresponding amino acid residues are aligned. For example, "valine residue (V) which is an amino acid residue at the 20th position from the N-terminus" in the amino acid sequence set forth in SEQ ID NO: 1 may be located at a position other than the 20th position from the N-terminus on the amino acid sequence to be compared. In the present disclosure, the "amino acid residue corresponding to the Xth position from the N-terminus of SEQ ID NO: 1" refers to an amino acid residue corresponding to the amino acid residue at the Xth position from the N-terminus of SEQ ID NO: 1 in the comparison target on the alignment.

Specifically, for example, valine, which is the amino acid residue at the 20th position from the N-terminus of SEQ ID NO: 1, may correspond to an amino acid residue at the 21st position from the N-terminus of the comparative target (which may not necessarily be a valine residue). In this case, the "amino acid residue corresponding to the 20th position from the N-terminus" in the description of the present disclosure refers to the amino acid residue which is the 21st position from the N-terminus in the amino acid sequence to be compared.

The method for producing a mutant glycosyltransferase according to the present disclosure is not particularly limited; and for example, the mutant glycosyltransferase can be produced by preparing a vector which contains a nucleic acid represented by a nucleotide sequence encoding the amino acid sequence of the mutant glycosyltransferase according to the present disclosure. Hereinafter, the details will be described.

<Nucleic acid>

The nucleic acid according to the present disclosure is a nucleic acid encoding the mutant glycosyltransferase according to the present disclosure. More specifically, the nucleic acid according to the present disclosure has a nucleotide sequence encoding the amino acid sequence of the mutant glycosyltransferase according to the present disclosure.

Examples of a method of synthesizing the nucleotide sequence encoding the amino acid sequence of the mutant glycosyltransferase according to the present disclosure include a method of introducing a mutation point into the nucleotide sequence encoding a corresponding wild-type glycosyltransferase derived from Punica granatum, and a method of chemically synthesizing an entire nucleotide sequence including the mutation point.

Examples of the method of causing a mutation in a gene using the nucleotide sequence encoding the wild-type glycosyltransferase derived from Punica granatum as a template include a site-specific mutagenesis method, a recombinant PCR method, a method of chemically synthesizing a specific portion of a nucleic acid, a method of treating a gene with hydroxylamine, a method of irradiating a strain carrying a gene with ultraviolet rays, a method of treating a strain with a chemical agent such as nitrosoguanidine and nitrous acid, and a method of using a commercially available mutation introduction kit.

The nucleic acid according to the present disclosure may be deoxyribonucleic acid (DNA) or ribonucleic acid (RNA).

<Vector>

The vector according to the present disclosure contains the nucleic acid according to the present disclosure.

Examples of the vector according to the present disclosure include a vector obtained by introducing the mutant glycosyltransferase according to the present disclosure into a known vector. The vector according to the present disclosure may be any of a virus vector, a phage vector, a plasmid vector, or the like.

The vector according to the present disclosure is preferably an expression vector.

The expression vector may be any expression vector as long as it contains the nucleic acid represented by the nucleotide sequence encoding the amino acid sequence of the mutant glycosyltransferase according to the present disclosure. With the expression vector according to the present disclosure, it is possible to produce the mutant glycosyltransferase according to the present disclosure by transforming any host cell to obtain a transformant or a cell strain, and then culturing the transformant or the cell strain.

The expression vector may include, as necessary, a nucleotide sequence (hereinafter, also simply referred to as "other regions") constituting a region, in addition to the nucleotide sequence encoding the mutant glycosyltransferase according to the present disclosure.

Examples of the other regions include a control region required for the above-described transformant to produce the mutant glycosyltransferase, and a region required for autonomous replication.

Examples of the above-described control region required for producing the mutant glycosyltransferase include a promoter sequence (including an operator sequence which controls transcription), a ribosome binding sequence (SD sequence), and a transcription termination sequence.

Examples of the promoter sequence include a trp promoter of a tryptophan operon derived from Escherichia coli, a lac promoter of a lactose operon, and a PL promoter or a PR promoter, which is derived from Lambda phage. In addition, a sequence which is artificially designed or modified, such as a tac promoter and a trc promoter, can also be used.

The sequence order of the above-described control region on the expression vector is not particularly limited, but for example, it is desirable that the promoter sequence and the ribosome binding sequence are positioned upstream of the gene encoding the mutant glycosyltransferase according to the present disclosure on the 5'-terminal side. In addition, it is desirable that the transcription termination sequence is located downstream of the gene encoding the mutant glycosyltransferase according to the present disclosure on the 3'-terminal side.

From the viewpoint of facilitating the selection of the above-described transformant, the expression vector may further include a nucleotide sequence encoding a selection gene which can be a selection marker.

A method of transforming a desired host cell with the expression vector and a method of causing the glycosyltransferase to be produced in the above-described transformant are not particularly limited, and a known general method or a host cell can be adopted.

<Host cell>

The host cell according to the present disclosure contains the expression vector according to the present disclosure.

With the host cell according to the present disclosure, a mutant glycosyltransferase having an excellent titer is produced.

Examples of a method of constructing the host cell according to the present disclosure include a method of constructing an expression vector including a nucleotide sequence encoding the mutant glycosyltransferase and, as necessary, the above-described other regions, and transforming a desired host cell with the expression vector.

Examples of the host cell include a prokaryote (such as Escherichia coli, Bacillus subtilis, and actinomycetes), yeast, and filamentous fungi; and among these, Escherichia coli is preferable.

Escherichia coli has a fast cell growth and has a lot of manufacturing experience at a low cost, and thus has excellent large-scale productivity.

The host cell (that is, the transformant) containing the expression vector may be modified, for example, by introducing a silent mutation such that a codon having a low frequency of use in the host cell is changed to a codon having a high frequency of use, as necessary.

<Method for producing mutant glycosyltransferase>

The method for producing a mutant glycosyltransferase according to the present disclosure is a method for producing a mutant glycosyltransferase including culturing the above-described host cell according to present disclosure in a culture medium, and recovering the mutant glycosyltransferase from at least one of the cultured host cell or the culture medium.

As conditions for culturing the host cell according to the present disclosure, that is, the host cell (also referred to as the transformant) transformed with the expression vector, known conditions can be used.

The culture medium may be any of a synthetic culture medium or a natural culture medium as long as it contains an appropriate amount of a carbon source, a nitrogen source, an inorganic substance, and other nutrients.

As the culture medium component, a known component used in the culture medium can be used. For example, an organic nutrient source such as meat extract, yeast extract, malt extract, peptone, NZ amine, and potato; a carbon source such as glucose, maltose, sucrose, starch, and organic acid; a nitrogen source such as ammonium sulfate, urea, and ammonium chloride; an inorganic nutrient source such as phosphate, magnesium, potassium, and iron; and vitamins can be used in appropriate combination. In addition, antibiotics such as kanamycin may be used for the selection of the transformant depending on the drug selection. In order to induce expression, an expression inducer such as an inducer of lac operon expression (isopropyl-β-thiogalactopyranoside or the like) may be used.

A pH of the culture medium can be appropriately selected, for example, in a range of pH 4 to pH 8.

A culture method for the transformant can be carried out using a general culture method such as shaking culture, aeration stirring culture, continuous culture, and feeding culture of a liquid culture medium containing the transformant.

The culture conditions may be appropriately selected depending on the type of the transformant, the culture medium, and the culture method; and the culture conditions are not particularly limited as long as the transformant grows and can produce the mutant glycosyltransferase.

The culture temperature is preferably 16°C to 45°C and more preferably 16°C to 40°C.

The culture period may be such that the content of the protein having a desired glycosyltransferase activity is maximized, for example, in a range of 10 hours to 7 days (preferably, in a range of 15 hours to 24 hours).

(Mutant glycosyltransferase recovery step)

The method for producing a mutant glycosyltransferase preferably includes a step of recovering the mutant glycosyltransferase from at least one of the cultured transformant or the culture medium after culture.

As a method of recovering the mutant glycosyltransferase after culturing the transformed host cell, a method which is commonly used in the field can be used.

For example, in a case where the mutant glycosyltransferase is secreted outside the transformant cell, a crude enzyme solution containing the mutant glycosyltransferase can be easily obtained by subjecting a culture of the transformant cell to centrifugation, filtration, or the like.

For example, in a case where the mutant glycosyltransferase is accumulated in the inside of the transformant cell, the transformant cell which has been cultured may be collected by centrifugation or the like, the collected transformant cell may be suspended in a buffer solution, and a cell membrane of the transformant cell in the suspension may be disrupted according to a known method such as lysozyme treatment, freeze-thawing, and ultrasonic disruption, whereby the crude enzyme solution containing the mutant glycosyltransferase may be collected.

In a case where further separation and purification are required, the recovered crude enzyme solution containing the mutant glycosyltransferase may be further separated and purified by, for example, a membrane separation method such as ultrafiltration, salting out, an organic solvent precipitation method, and dialysis; or a known chromatographic separation method such as ion exchange chromatography and gel filtration chromatography, which may be appropriately combined.

<Composition>

The composition according to the present disclosure is a composition containing the mutant glycosyltransferase according to the present disclosure. The composition according to the present disclosure has an excellent titer.

The composition according to the present disclosure may further contain other materials in addition to the mutant glycosyltransferase. Examples of the other materials include a cell debris of the transformant (for example, a cell membrane) generated in the process of producing the mutant glycosyltransferase, a buffer solution, a solvent, and a culture solution containing a culture component.

<Method for producing anthraquinone C-glycoside>

The method for producing an anthraquinone C-glycoside according to the present disclosure is a method for producing an anthraquinone C-glycoside comprising a step of bringing an anthraquinone compound and a monosaccharide donor into contact with the mutant glycosyltransferase according to the present disclosure to form an anthraquinone C-glycoside.

According to the method for producing an anthraquinone C-glycoside according to the present disclosure, efficient production of an anthraquinone C-glycoside tends to be possible.

The anthraquinone compound is not particularly limited as long as it has an anthraquinone skeleton and can act as a sugar receptor in a glycosyl transfer reaction by glycosyltransferase, and known anthraquinone compounds can be adopted. Examples of the anthraquinone compound include kermesic acid, flavokermesic acid, emodin, 2-methylanthraquinone, 1-chloroanthraquinone, and 2-amylanthraquinone.

The monosaccharide donor is not particularly limited as long as it can act as a donor of a monosaccharide in a glycosyl transfer reaction by the glycosyltransferase, and examples thereof include a nucleotide sugar such as glucose, fructose, and galactose. Examples of the nucleotide sugar include uridine diphosphate glucose.

In particular, the mutant glycosyltransferase according to the present disclosure is excellent in a reaction of forming an anthraquinone C-glycoside containing a cochineal coloring agent from an anthraquinone compound containing at least one of kermesic acid or flavokermesic acid and a glucose donor (for example, uridine diphosphate glucose) as a monosaccharide donor.

The method of bringing the anthraquinone compound and the monosaccharide donor into contact with the mutant glycosyltransferase according to the present disclosure is not particularly limited; and for example, an aspect in which the anthraquinone compound and the monosaccharide donor are mixed with a culture solution, a cell-treated product, or an enzyme purification product, which contains the transformant or the cell strain producing the mutant glycosyltransferase according to the present disclosure may be used.

As a result, the mutant glycosyltransferase according to the present disclosure comes into contact with the anthraquinone compound and the monosaccharide donor, and catalyzes a reaction of forming an anthraquinone C-glycoside from the anthraquinone compound and the monosaccharide donor.

The above-described cell-treated product refers to, for example, an extract or a cell debris from the transformant.

The temperature during the above-described contact and reaction is, for example, preferably 10°C to 30°C and more preferably 15°C to 30°C.

As one aspect, the anthraquinone C-glycoside may be formed by mixing a purified product of the mutant glycosyltransferase according to the present disclosure, which is extracted and purified from a culture solution in which the transformant or the cell strain is cultured, with a solvent containing at least the anthraquinone compound and the monosaccharide donor, and bringing the two into contact with each other to react.

A pH of the aqueous solution in a case of the above-described contact and reaction is preferably 6 to 8 and more preferably 7 to 8.

Details of the culture of the transformant are as described in the section of the method for producing a mutant glycosyltransferase according to the present disclosure.

Examples

Hereinafter, the embodiments will be described in more detail with reference to Examples, but the present invention is not limited to the following examples.

1. Acquisition of wild-type mutant glycosyltransferase

<Comparative Example 1: Acquisition of comparative mutant glycosyltransferase (c1)>

A mutant glycosyltransferase (c1) was obtained by the method disclosed in Dongsoo Yang, Woo Dae Jang, Sang Yup Lee, Journal of American Chemical Society, 2021, 143, 14, pp. 5364 to 5377.

<Comparative Example 2: Acquisition of comparative mutant glycosyltransferase (c2)>

A mutant glycosyltransferase (c2) was obtained by the method disclosed in Dongsoo Yang, Woo Dae Jang, Sang Yup Lee, Journal of American Chemical Society, 2021, 143, 14, pp. 5364 to 5377.

<Comparative Example 3: Acquisition of comparative mutant glycosyltransferase (c3)>

Since a mutant glycosyltransferase (c3) disclosed in US10100290B is disclosed as being difficult to express in Escherichia coli (that is, having a low titer), the mutant glycosyltransferase (c3) was not acquired in the present disclosure; and the evaluations of the titer, the C-glycosylation activity, and the C-glycosylation activity which can be extracted from a culture solution were not carried out.

<Reference Example 1: Acquisition of wild-type glycosyltransferase (r1) derived from Punica granatum>

A wild-type glycosyltransferase (r1) derived from Punica granatum was obtained by a method described in the document (Nadia N. Ono, Xiaoqiong Qin, Alexander E. Wilson, Gang Li1, Li Tian, PLoS ONE, 11(5): e0156319).

An amino acid sequence (SEQ ID NO: 72) of the mutant glycosyltransferase (c1) in Comparative Example 1, an amino acid sequence (SEQ ID NO: 73) of the mutant glycosyltransferase (c2) in Comparative Example 2, an amino acid sequence (SEQ ID NO: 74) of the mutant glycosyltransferase (c3) in Comparative Example 3, and an amino acid sequence (SEQ ID NO: 75) of the wild-type glycosyltransferase (r1) derived from Punica granatum in Reference Example 1 are shown below.

SEQ ID NO: 72:

MGSLTNNDNLHIFLVCFIGQGVVNPMLRLGKAFASKGLLVTLSAPEIVGTEIRKANNL
NDDQPIKVGSGMIRFEFFDDGWESVNGSKPFDVWVYINHLDQTGRQKLPIMLKKHEE
TGTPVSCLILNPLVPWVADVADSLQIPCATLWVQSCASFSAYYHYHHGLVPFPTESEP
EIDVQLPGMPLLKYDEVPDYLHPRTPYPFFGTNILGQFKNLSKNFCILMDTFYELEHEII
DNMCKLCPIKPIGPLFKIPKDPSSNGITGNFMKVDDCKEWLDSRPTSTVVYVSVGSVV
YLKQEQVTEMAYGILNSEVSFLWVLRPPSKRIGTEPHVLPEEFWEKAGDRGKVVQWS
PQEQVLAHPATVGFLTHCGWNSTQEAISSGVPVITFPQFGDQVTNAKFLVEEFKVGVR
LGRGELENRIITRDEVERALREITSGPKAEEVKENALKWKKKAEETVAKGGYSERNLV
GFIEEVARKTGTK

SEQ ID NO: 73:

MGSLTNNDNLHIFLVCFIGQGVVNPMLRLGKAFASKGLLVTLSAPEIVGTEIRKANNL
NDDQPIKVGSGMIRFEFFDDGWESVNGSKPFDVWQYINHLDQTGRQKLPIMLKKHEE
TGTPVSCLILNPLVPWVADVADSLQIPCATLWVQSCASFSAYYHYHHGLVPFPTESEP
EIDVQLPGMPLLKYDEVPDFLHPRTPYPFFGTNILGQFKNLSKNFCILMDTFYELEHEII
DNMCKLCPIKPIGPLFKIPKDPSSNGITGNFMKVDDCKEWLDSRPTSTVVYVSVGSVV
YLKQEQVTEMAYGILNSEVSFLWVLRPPSKRIGTEPHVLPEEFWEKAGDRGKVVQWS
PQEQVLAHPATVGFLTHCGWNSTQEAISSGVPVITFPQFGDQVTNAKFLVEEFKVGVR
LGRGELENRIITRDEVERALREITSGPKAEEVKENALKWKKKAEETVAKGGYSERNLV
GFIEEVARKTGTK

SEQ ID NO: 74:

MEFRLLILALFSVLMSTSNGAEILALFPIHGISNYNVAEALLKTLANRGHNVTVVTSFP
QKKPVPNLYEIDVSGAKGLATNSIHFERLQTIIQDVKSNFKNMVRLSRTYCEIMFSDPR
VLNIRDKKFDLVINAVFGSDCDAGFAWKSQAPLISILNARHTPWALHRMGNPSNPAY
MPVIHSRFPVKMNFFQRMINTGWHLYFLYMFYYGNGEDANKMARKFFGNDMPDI
NEMVFNTSLLFVNTHFSVDMPYPLVPNCIEIGGIHVKEPQPLPLEIQKFMDEAEHGVIF
FTLGSMVRTSTFPNQTIQAFKEAFAELPQRVLWKFENENEDMPSNVLIRKWFPQNDIF
GHKNIKAFISHGGNSGALEAVHFGVPIIGIPLFYDQYRNILSFVKEGVAVLLDVNDLTK
DNILSSVRTVVNDKSYSERMKALSQLFRDRPMSPLDTAVYWTEYVIRHRGAHHLKTA
GAFLHWYQYLLLDVITFLLVTFCAFCFIVKYICKALIHHYWSSSKSEKLKKN

SEQ ID NO: 75:

MGSESSLVHVFLVSFPGQGHVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDSGYTVLTLPEGFLEKAGDRGKVVQWSPQE
QVLAHPATACFVTHCGWNSSMEALTSGMPVVAFPQWGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

2. Acquisition of mutant glycosyltransferase according to present disclosure

<Acquisition of mutant glycosyltransferase (1) according to present disclosure>

A nucleic acid encoding a polypeptide of SEQ ID NO: 1 was subjected to codon optimization and synthesized as a nucleic acid gene. Competent cells of an Escherichia coli strain BL21 (DE3) were transformed with a pET28a vector system in which the synthetic gene was cloned, and the cells were seeded on an LB agar medium containing 50 μg/mL of kanamycin and cultured overnight at 37°C. The proliferated colonies were inoculated in an LB liquid medium containing 50 μg/mL of kanamycin, and subjected to overnight shaking culture at 37°C and 200 rpm. A part of the culture solution was inoculated into 0.3 L of an LB liquid medium containing 50 μg/mL of kanamycin, and cultured at 37°C and 200 rpm. After an optical density (OD600) at 600 nm reached 0.6 to 0.8, isopropyl-β-thiogalactopyranoside (IPTG) was added thereto to a final concentration of 0.5 mM, and shaking culture was carried out at 16°C and 200 rpm for 18 hours. The supernatant was discarded by centrifugation (4°C, 3,000 G, 20 min). The obtained cell pellet was suspended in 30 mL of a lysis buffer (1 × PBS, 10 mM imidazole solution (pH: 7.4)) and treated on ice using a homogenizer (manufactured by BRANSON SONIFER). The treatment liquid was centrifuged (4°C, 5,000 G, 30 min) to discard cell debris. The obtained supernatant was subjected to His tag purification, subjected to ultrafiltration, and replaced with a 50 mM HEPES buffer (pH 7.5) to obtain a purified enzyme solution, thereby obtaining a composition containing the mutant glycosyltransferase. A sequence of the above-described nucleic acid gene (SEQ ID NO: 76) is shown below.

SEQ ID NO: 76:

ATGGGCAGCGAAAGCAGCCTGGTGCATGTGTTTCTGGTGAGCTTTCCGGGCCAAG

GCGTGGTGAACCCGCTGCTGCGCCTGGGCAAACGCCTGGCGAGCAAAGGCCTGCT

GGTGACCTTTACCACCCCGGAAAGCATTGGCAAACAGATGCGCAAAGCGAGCAA

CATTAGCGATCAGCCGGCGCCGGTGGGCGATGGCTTTATTCGCTTTGAATTTTTG

AAGATGGCTGGGATGAAGATGAACCGCGCCGCCAAGATCTGGATCAGTATCTGCC

GCAGCTGGAAAAGTGGGCAAAGTGCTGATTCCGCAGATGATTCAGAAAACGC

GGAACAAGGCCGCCCGGTGAGCTGCCTGATTAACAACCCGTTTATTCGTGGGTG

AGCGATGTGGCGGAAACCCTGGGCCTGCCGAGCGCGATGCTGTGGGTGCAGAGCT

GCGCGTGCTTTCTGGCGTATTATCATTATTATCATGGCCTGGTGCCGTTTCCGAGC

GAAAACGCGATGGAAATTGATGTGCAGCTGCCGAGCATGCCGCTGCTGAAACATG

ATGAAGTGCCGAGCTTTCTGTATCCGACCACCCCGTATCCGTTTCTGCGCCGCGCG

ATTCTGGGTCAGTATAAAAACCTGGAAAAACCGTTTTGCATTCTGATGGATACCTT

TCAAGAACTGGAACATGAAATTATTGAATATCGAGCAAAATTTGCCCGATTAAA

ACCGTGGGCCCGCTGTTTAAAAACCCGAAAGCGCCGAACACCACCGTGAAAGGC

GATTTTATGAAAGCGGATGATTGCATTGGCTGGCTGGATAGCAAGCCGGCTAGCT

CTGTGGTGTACGTATCTTTTGGCAGTGTGGTTTACCTGAAACAAGATCAGTGGGAT

GAAATTGCGTATGGCCTGCTGAACAGCGGCGTGAACTTTCTGTGGGTGATGAAAC

CGCCGCATAAAGATAGCGGCTATACCGTGCTGACCCTGCCGGAAGGCTTTCTGGA

AAAAGCGGGCGATCGCGGCAAAGTGGTGCAGTGGAGCCCGCAAGAACAAGTGCT

GGCGCATCCGGCGACCGCGTGCTTTGTGACCCATTGCGGCTGGAACAGCAGCATG

GAAGCGCTGACGAGCGGCATGCCGGTGGTGGCGTTTCCGCAGTGGGGCGATCAAG

TGACCGATGCGAAATATCTGGTGGATGAATTTAAAGTGGGCGTGCGCATGTGCCG

CGGCGAAGCGGAAGATAAACTGATTACCCGCGATGTGGTGGAACAGTGCCTGCGC

GAAGCGACCCAAGGCCCGAAAGCGGCGGAAATGAAAAAAACGCGCTGAAATGG

AAAGCTGCGGCTGAGGCGAGCTTTGTGGAAGGCGGCAGCAGCGATCGCAACCTG

CAAGCGTTTGTGGATGAAGTGAAACGCCGCAGCATTGAAATTACTGCGAGCAAAC

CGGCGGTCAAAGCGGCGCCGAATGGCGTGGTGGCGGCGGCCGAGAGCGTGGTGG

AAACCAAAGCGAACGGCAAAGTGGAACTGGCGGCGTAA

<Acquisition of mutant glycosyltransferases (2) to (71) according to present disclosure>

A nucleic acid encoding a polypeptide having SEQ ID NO: represented in the tables below (referred to as "Enzyme sequence number" in the tables) was subjected to codon optimization and synthesized as a gene. In addition, in the same manner as in the case of the mutant glycosyltransferase (1), compositions containing mutant glycosyltransferases (2) to (71) were obtained.

In the tables described below, the notation of one capital letter in the items of [Mutation 1 G17] to [Mutation 6 I46] indicates the type of the corresponding amino acid residue in the amino acid sequence set forth in SEQ ID NO: 1. For example, the item of [Mutation 1 G17] in Table 3 refers to a mutation relating to a glycine residue (G) which is an amino acid residue at the 17th position from the N-terminal side in the amino acid sequence set forth in SEQ ID NO: 1.

In the tables below, the blank portions in the items of [Mutation 1 G17] to [Mutation 6 I46] indicate that the amino acid residues of each item were not mutated from the amino acid residue set forth in SEQ ID NO: 1.

In the tables below, "H*" described in the item of [Mutation 2 V20] of Reference Example 1 indicates that the 20th amino acid residue from the N-terminal side in the amino acid sequence of the wild-type glycosyltransferase derived from Punica granatum was a histidine residue (H).

The mutant glycosyltransferase (2) was a mutant glycosyltransferase having no mutations at G17, V20, S318, Y320, Y382, and I46 with respect to SEQ ID NO: 1, but having mutations in other amino acids, as a result, the amino acid sequence identity with respect to SEQ ID NO: 1 was 85%. The mutant glycosyltransferases (3) to (71) had mutations at G17, V20, S318, Y320, Y382, and I46 with respect to SEQ ID NO: 1, but did not have mutations at the other positions.

3. Comparison of titer and activity of glycosyltransferase

Using the acquired glycosyltransferases, the titer and the enzyme activity were compared.

[Evaluation of titer (a)]

For the purified enzyme solution of each example, an enzyme amount (that is, an amount of the mutant glycosyltransferase) was quantified from a concentration of the purified enzyme solution based on the absorbance at 280 nm measured with a spectrophotometer. Subsequently, the enzyme diluted solution was developed by SDS-PAGE, the gel is stained with a Coomassie Brilliant Blue (CBB) staining solution, and then the purity of a band containing a target protein (that is, a band corresponding to the mutant glycosyltransferase) was quantified by image analysis. From the acquired enzyme amount and the culture solution amount, the titer was calculated based on the following expression, and evaluated according to the following standard. The results are shown in the table below.

Enzyme amount (mg) = Enzyme solution concentration (mg/L) × Enzyme solution amount (L) × (Purity (%)/100)

$$\text{Titer (mg/L)} = \text{Enzyme amount (mg)/Culture solution amount (L)}$$

-Evaluation standard-

S: titer was 10 (mg/L) or more.
A: titer was 3 (mg/L) or more and less than 10 (mg/L).
B: titer was 1 (mg/L) or more and less than 3 (mg/L).
C: titer was less than 1 (mg/L).

[Evaluation of C-glycosylation activity (b)]

The evaluation of the C-glycosylation activity (b) in a case where kermesic acid was used as a substrate was carried out according to the following specifications.

A DMSO solution of 0.5 mM of kermesic acid, an aqueous solution of 5 mM of uridine diphosphate glucose (UDP-Glucose), and 20 μg of the purified enzyme of each example were stirred and reacted at 25°C for 3 hours in 100 μL of a buffer (mixed solution of 50 mM of HEPES (pH: 7.5), 1 mg/mL of lysozyme, 0.5 mg/mL of polymyxin B, 13 mM of $MgCl_2$, and 50 mM of KCl). Thereafter, 100 μL of methanol was added to the reaction system to terminate the reaction, and centrifugation (4°C, 1,800 G, 30 min) was carried out to obtain a supernatant.

The supernatant was subjected to high performance liquid chromatography (HPLC) measurement, and the amount of the target product, anthraquinone C-glycoside, was quantitatively analyzed. The HPLC analysis conditions were as follows.

-HPLC analysis conditions-

Device: Nexera X2 (manufactured by Shimadzu Corporation)
Column: Waters ACQUITY UPLC BEH C18, 1.7 μm, 2.0 × 50 mm
Mobile phase: A: water (0.1% formic acid), B: methanol (0.1% formic acid)
Time (min)/%B: 0/10, 0.4/10, 3.0/80, 4.0/100, 5.0/100, 5.1/10
Injection volume: 2 μL
Column temperature: 40°C
Flow rate: 0.4 mL/min
Detection wavelength: 490 nm for kermesic acid
Retention time: 2.34 min for Kermes acid C-glycoside

The C-glycosylation activity was calculated from the amount of the anthraquinone C-glycoside produced, the reaction time, and the amount of enzyme used by HPLC, and evaluated according to the following standard. The results are shown in the tables below.

C-glycosylation activity (pkat/mg) = Amount of produced C-glycoside (pmol)/Reaction time (s)/Amount of enzyme used (mg)

-Evaluation standard-

S: C-glycosylation activity was 100 (pkat/mg) or more.
A: C-glycosylation activity was 10 (pkat/mg) or more and less than 100 (pkat/mg).
B: C-glycosylation activity was 1 (pkat/mg) or more and less than 10 (pkat/mg).
C: C-glycosylation activity was less than 1 (pkat/mg).

[Evaluation of C-glycosylation activity (a × b) which could be extracted from culture solution]

From the C-glycosylation activity and the titer obtained by the above-described evaluations, the enzyme activity which could be extracted from the culture solution was calculated based on the following expression, and evaluated according to the following standard. The results are shown in the tables below.

Enzyme activity (pkat/L) which could be extracted from culture solution = Anthraquinone C-glycosylation activity (pkat/mg) × Expression efficiency (mg/L)

-Evaluation standard-

S: enzyme activity which could be extracted from the culture solution was 1,000 (pkat/L) or more.
A: enzyme activity which could be extracted from the culture solution was 100 (pkat/L) or more and less than 1,000 (pkat/L).
B: enzyme activity which could be extracted from the culture solution was 10 (pkat/L) or more and less than 100 (pkat/L).
C: enzyme activity which could be extracted from the culture solution was less than 10 (pkat/L).

[Table 6]

| | Enzyme sequence number | Mutation 1 (G17) | Mutation 2 (V20) | Mutation 3 (S318) | Mutation 4 (Y320) | Mutation 5 (W382) | Mutation 6 (I46) | Sequence identity to SEQ ID NO:1 | Titer (a) | C-glycosylation activity (b) | C-glycosylation activity which could be extracted from culture solution (a × b) |
|---|---|---|---|---|---|---|---|---|---|---|---|
| Comparative Example 1 | 72 | F | Q | P | S | I | I | 59% | C | B | C |
| Comparative Example 2 | 73 | F | Q | P | S | I | I | 59% | C | A | B |
| Comparative Example 3 | 74 | T | G | Q | v | D | A | 17% | - | - | - |
| Reference Example 1 | 75 | | H* | | | | | 99% | S | C | C |
| Example 1 | 1 | | | | | | | (Standard) 100% | S | C | B |
| Example 2 | 2 | | | | | | | 85% | S | C | C |
| Example 3 | 3 | H | | | | | | 99% | S | C | B |
| Example 4 | 4 | S | | | | | | 99% | S | B | B |
| Example 5 | 5 | | C | | | | | 99% | S | B | B |
| Example 6 | 6 | H | C | | | | | 99% | S | B | B |
| Example 7 | 7 | H | N | | | | | 99% | S | B | B |
| Example 8 | 8 | H | I | | | | | 99% | S | C | B |
| Example 9 | 9 | S | C | | | | | 99% | S | B | B |
| Example 10 | 10 | S | N | | | | | 99% | S | B | A |
| Example 11 | 11 | S | I | | | | | 99% | S | B | B |
| Example 12 | 12 | | | R | | | | 99% | S | B | B |
| Example 13 | 13 | | | | H | | | 99% | S | C | B |
| Example 14 | 14 | | | | R | | | 99% | S | B | B |
| Example 15 | 15 | | | | v | | | 99% | S | C | B |
| Example 16 | 16 | | | H | R | | | 99% | S | C | B |

EP 4 682 259 A1

(continued)

| | Enzyme sequence number | Mutation 1 (G17) | Mutation 2 (V20) | Mutation 3 (S318) | Mutation 4 (Y320) | Mutation 5 (W382) | Mutation 6 (I46) | Sequence identity to SEQ ID NO:1 | Titer (a) | C-glycosylation activity (b) | C-glycosylation activity which could be extracted from culture solution (a × b) |
|---|---|---|---|---|---|---|---|---|---|---|---|
| Example 17 | 17 | | | H | v | | | 99% | S | B | B |
| Example 18 | 18 | | | R | H | | | 99% | S | B | B |
| Example 19 | 19 | | | R | R | | | 99% | S | B | B |
| Example 20 | 20 | | | R | C | | | 99% | S | C | B |

[Table 7]

| | Enzyme sequence number | Mutation 1 (G17) | Mutation 2 (V20) | Mutation 3 (S318) | Mutation 4 (Y320) | Mutation 5 (W382) | Mutation 6 (I46) | Sequence identity to SEQ ID NO:1 | Titer (a) | C-glycosylation activity (b) | C-glycosylation activity which could be extracted from culture solution (a × b) |
|---|---|---|---|---|---|---|---|---|---|---|---|
| Example 21 | 21 | | | R | S | | | 99% | S | C | B |
| Example 22 | 22 | | | R | I | | | 99% | S | B | B |
| Example 23 | 23 | | | R | v | | | 99% | S | B | B |
| Example 24 | 24 | | | N | v | | | 99% | S | C | B |
| Example 25 | 25 | | | G | R | | | 99% | S | C | B |
| Example 26 | 26 | | | I | R | | | 99% | S | B | B |
| Example 27 | 27 | | | I | I | | | 99% | S | B | B |
| Example 28 | 28 | | | I | v | | | 99% | S | B | B |
| Example 29 | 29 | | | L | R | | | 99% | S | C | B |
| Example 30 | 30 | | | v | R | | | 99‰ | S | C | B |
| Example 31 | 31 | | | v | I | | | 99% | S | B | B |
| Example 32 | 32 | | | V | L | | | 99% | S | C | B |
| Example 33 | 33 | | | V | V | | | 99% | S | C | B |
| Example 34 | 34 | | | F | R | | | 99% | S | B | B |
| Example 35 | 35 | | | F | I | | | 99% | S | B | B |
| Example 36 | 36 | | | F | V | | | 99% | S | B | B |
| Example 37 | 37 | | | Y | I | | | 99% | S | B | B |
| Example 38 | 38 | | | Y | v | | | 99% | S | C | B |
| Example 39 | 39 | | | | | Y | | 99% | S | B | B |
| Example 40 | 40 | S | N | | | Y | | 99% | S | A | S |

EP 4 682 259 A1

[Table 8]

| | Enzyme sequence number | Mutation 1 (G17) | Mutation 2 (V20) | Mutation 3 (S318) | Mutation 4 (Y320) | Mutation 5 (W382) | Mutation 6 (I46) | Sequence identity to SEQ ID NO:1 | Titer (a) | C-glycosylation activity (b) | C-glycosylation activity which could be extracted from culture solution (a × b) |
|---|---|---|---|---|---|---|---|---|---|---|---|
| Example 41 | 41 | S | N | | | Y | C | 99% | S | A | S |
| Example 42 | 42 | S | N | | | Y | s | 99% | S | A | S |
| Example 43 | 43 | S | N | | | Y | G | 99% | S | A | S |
| Example 44 | 44 | S | N | | | Y | v | 99% | S | A | S |
| Example 45 | 45 | S | N | | | Y | F | 99% | S | A | S |
| Example 46 | 46 | S | N | | | Y | Y | 99% | S | A | S |
| Example 47 | 47 | S | N | R | | Y | F | 99% | S | S | S |
| Example 48 | 48 | S | N | v | | Y | F | 99% | S | S | S |
| Example 49 | 49 | S | N | | R | Y | F | 99% | S | S | S |
| Example 50 | 50 | S | N | | v | Y | F | 99% | S | A | S |
| Example 51 | 51 | S | N | H | R | Y | F | 99% | S | S | S |
| Example 52 | 52 | S | N | H | v | Y | F | 99% | S | S | S |
| Example 53 | 53 | S | N | R | H | Y | F | 99% | S | A | S |
| Example 54 | 54 | S | N | R | R | Y | F | 99% | S | S | S |
| Example 55 | 55 | S | N | R | D | Y | F | 99% | S | S | S |
| Example 56 | 56 | S | N | R | C | Y | F | 99% | S | S | S |
| Example 57 | 57 | S | N | R | G | Y | F | 99% | S | S | S |
| Example 58 | 58 | S | N | R | I | Y | F | 99% | S | S | S |
| Example 59 | 59 | S | N | R | v | Y | F | 99% | S | S | S |

[Table 9]

| | Enzyme sequence number | Mutation 1 (G17) | Mutation 2 (V20) | Mutation 3 (S318) | Mutation 4 (Y320) | Mutation 5 (W382) | Mutation 6 (I46) | Sequence identity to SEQ ID NO:1 | Titer (a) | C-glycosyl ation activity (b) | C-glycosyla tion activity which could be extracted from culture solution (a × b) |
|---|---|---|---|---|---|---|---|---|---|---|---|
| Example 61 | 61 | S | N | C | R | Y | F | 99% | S | S | S |
| Example 62 | 62 | S | N | G | R | Y | F | 99% | S | S | S |
| Example 63 | 63 | S | N | I | R | Y | F | 99% | S | S | S |
| Example 64 | 64 | S | N | I | v | Y | F | 99% | S | S | S |
| Example 65 | 65 | S | N | L | v | Y | F | 99% | S | S | S |
| Example 66 | 66 | S | N | v | R | Y | F | 99% | S | S | S |
| Example 67 | 67 | S | N | v | L | Y | F | 99% | S | S | S |
| Example 68 | 68 | S | N | v | v | Y | F | 99% | S | S | S |
| Example 69 | 69 | S | N | v | F | y | F | 99% | S | S | S |
| Example 70 | 70 | S | N | F | R | Y | F | 99% | S | S | S |
| Example 71 | 71 | S | N | F | v | Y | F | 99% | S | A | S |

As shown in the above tables, it was found that the mutant glycosyltransferases of Examples had an excellent titer as compared with the wild-type or mutant glycosyltransferases of Comparative Examples. In addition, a mutant glycosyltransferase having improved C-glycosylation activity was also acquired by introducing a mutation into SEQ ID NO: 1. In the acquired mutant glycosyltransferase in this manner, particularly, the mutant glycosyltransferases of Examples, in which the sequence identity with SEQ ID NO: 1 was 90% or more, tended to have a favorable C-glycosylation activity which could be extracted from the culture solution.

4. Evaluation of C-glycosylation activity with respect to flavokermesic acid

Next, the C-glycosylation activity using flavokermesic acid as a substrate was evaluated instead of kermesic acid.

[Evaluation of C-glycosylation activity (b)]

The evaluation of the C-glycosylation activity (b) was carried out in the same manner as in Example 1, except that the reaction step in the evaluation of the C-glycosylation activity (b) in a case where the flavocermic acid was used as a substrate was carried out according to the following specifications, the detection wavelength in the HPLC analysis was set to a wavelength of 430 nm for flavokermesic acid, and the retention time was set to a time of 2.29 min for flavokermesic acid C-glycoside. The results of each evaluation are shown in the table below.

A DMSO solution of 0.5 mM of flavokermesic acid, an aqueous solution of 5 mM of uridine diphosphate glucose (UDP-Glucose), and 20 $\mu$g of the purified enzyme of each example were stirred and reacted at 25°C for 24 hours in 100 $\mu$L of a buffer (mixed solution of 50 mM of HEPES (pH: 7.5), 1 mg/mL of lysozyme, 0.5 mg/mL of polymyxin B, 13 mM of $MgCl_2$, and 50 mM of KCl). Thereafter, 100 $\mu$L of methanol was added to the reaction system to terminate the reaction, and centrifugation (4°C, 1,800 G, 30 min) was carried out to obtain a supernatant.

[Evaluation of C-glycosylation activity (a $\times$ b) which could be extracted from culture solution]

The C-glycosylation activity (a $\times$ b) which could be extracted from the culture solution using the mutant glycosyltransferase of each example was evaluated by the same method as in Example 1 described above. The results are shown in the table below.

In the table, the titer is a titer measured by the above-described method.

[Table 10]

| | Enzyme sequence number | Mutation 1 (G17) | Mutation 2 (V20) | Mutation 3 (S318) | Mutation 4 (Y320) | Mutation 5 (W382) | Mutation 6 (146) | Sequence identity to SEQ ID NO:1 | Titer (a) | C-glycosylation activity (b) | C-glycosylatio n activity which could be extracted from culture solution (a × b) |
|---|---|---|---|---|---|---|---|---|---|---|---|
| Example 1-2 | 1 | | | | | | | (Standard) 100% | S | C | C |
| Example 10-2 | 10 | S | N | | | | | 99% | S | B | B |
| Example 40-2 | 40 | s | N | | | Y | | 99% | S | B | B |
| Example 45-2 | 45 | s | N | | | Y | F | 99% | S | B | B |
| Example 59-2 | 59 | S | N | R | V | Y | F | 99% | S | B | A |
| Example 70-2 | 70 | S | N | F | R | Y | F | 99% | S | B | A |
| Example 71-2 | 71 | s | N | F | v | Y | F | 99% | S | B | B |

From the above table, it was found that each variant also had an excellent C-glycosylation activity with respect to flavokermesic acid.

5. Examination of C-glycosylation activity with respect to emodin

Next, the C-glycosylation activity using emodin as a substrate was evaluated instead of kermesic acid.

[Evaluation of C-glycosylation activity (b)]

The evaluation of the C-glycosylation activity (b) was carried out in the same manner as in Example 1, except that the reaction step in the evaluation of the C-glycosylation activity (b) in a case where the emodin (6-methyl-1,3,8-trihydroxyanthraquinone) was used as a substrate was carried out according to the following specifications, the detection wavelength in the HPLC analysis was set to a wavelength of 420 nm for emodin, and the retention time was set to a time of 3.14 min for emodin C-glycoside. The results of each evaluation are shown in the table below.

A DMSO solution of 0.5 mM of emodin, an aqueous solution of 5 mM of uridine diphosphate glucose (UDP-Glucose), and 20 $\mu$g of the purified enzyme of each example were stirred and reacted at 25°C for 24 hours in 100 $\mu$L of a buffer (mixed solution of 50 mM of HEPES (pH: 7.5), 1 mg/mL of lysozyme, 0.5 mg/mL of polymyxin B, 13 mM of $MgCl_2$, and 50 mM of KCl). Thereafter, 100 $\mu$L of methanol was added to the reaction system to terminate the reaction, and centrifugation (4°C, 1,800 G, 30 min) was carried out to obtain a supernatant.

[Evaluation of C-glycosylation activity (a $\times$ b) which could be extracted from culture solution]

The C-glycosylation activity (a $\times$ b) which could be extracted from the culture solution using the mutant glycosyltransferase of each example was evaluated by the same method as in Example 1 described above. The results are shown in the table below.

In the table, the titer is a titer measured by the above-described method.

[Table 11]

| | Enzyme sequence number | Mutation 1 (G17) | Mutation 2 (V20) | Mutation 3 (S318) | Mutation 4 (Y320) | Mutation 5 (W382) | Mutation 6 (I46) | Sequence identity to SEQ ID NO:1 | Titer (a) | C-glycosylation activity (b) | C-glycosylation activity which could be extracted from culture solution (a × b) |
|---|---|---|---|---|---|---|---|---|---|---|---|
| Example 1-3 | 1 | | | | | | | (Standard) 100% | S | C | C |
| Example 10-3 | 10 | S | N | | | | | 99% | S | C | C |
| Example 40-3 | 40 | S | N | | | Y | | 99% | S | C | B |
| Example 45-3 | 45 | S | N | | | Y | F | 99% | S | C | B |
| Example 59-3 | 59 | S | N | R | v | Y | F | 99% | S | C | C |
| Example 70-3 | 70 | S | N | F | R | Y | F | 99% | S | C | B |
| Example 71-3 | 71 | S | N | F | v | Y | F | 99% | S | C | B |

From the above table, it was found that each variant also had an excellent C-glycosylation activity with respect to emodin.

The amino acid sequence of the mutant glycosyltransferase of each example is shown below.

SEQ ID NO: 1:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDSGYTVLTLPEGFLEKAGDRGKVVQWSPQE
QVLAHPATACFVTHCGWNSSMEALTSGMPVVAFPQWGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 2:

MGSESLVHVFLVSFPGQGVVNPLLRLGKRLASKGLLVTFTTPESIGKQMRKASNIGEE
PSPIGDGFIRFEFFEDGWDEDEPRRQDLDQYLPQLEKVGKEVIPRMIKKNEEQNRPVSC
LINNPFIPWVSDVAESLGLPSAMLWVQSCACFAAYYHYYHGLVPFPSESAMEIDVQLP
CMPLLKHDEVPSFLYPTTPYPFLRRAIMGQYKNLDKPFCVLMDTFQELEHEIIEYMSKI
CPIKTVGPLFKNPKAPNANVRGDFMKADDCISWLDSKPPASVVYVSFGSVVYLKQDQ
WDEIAFGLLNSGLNFLWVMKPPHKDSGYQLLTLPEGFLEKAGDKGKVVQWSPQEQV
LAHPSVACFVTHCGWNSSMEALSSGMPVVAFPQWGDQVTDAKYLVDVFKVGVRMC
RGEAENKLIMRDVVEKCLLEATVGPKAAEVKENALKWKAAAEAAVAEGGSSDRNIQ
AFVDEVKRRSIAIQSNKSEPKPVVQNAAVADHFGAKATTNGVAADLAGSNADGKVE
LVA

SEQ ID NO: 3:

MGSESSLVHVFLVSFPHQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDSGYTVLTLPEGFLEKAGDRGKVVQWSPQE
QVLAHPATACFVTHCGWNSSMEALTSGMPVVAFPQWGDQVTDAKYLVDEFKVGVR

MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 4:

MGSESSLVHVFLVSFPSQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDSGYTVLTLPEGFLEKAGDRGKVVQWSPQE
QVLAHPATACFVTHCGWNSSMEALTSGMPVVAFPQWGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 5:

MGSESSLVHVFLVSFPGQGCVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDSGYTVLTLPEGFLEKAGDRGKVVQWSPQE
QVLAHPATACFVTHCGWNSSMEALTSGMPVVAFPQWGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 6:

MGSESSLVHVFLVSFPHQGCVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDSGYTVLTLPEGFLEKAGDRGKVVQWSPQE
QVLAHPATACFVTHCGWNSSMEALTSGMPVVAFPQWGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 7:

MGSESSLVHVFLVSFPHQGNVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDSGYTVLTLPEGFLEKAGDRGKVVQWSPQE
QVLAHPATACFVTHCGWNSSMEALTSGMPVVAFPQWGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 8:

MGSESSLVHVFLVSFPHQGIVNPLLRLGKRLASKGLLVTFTTPESIGKQMRKASNISDQ
PAPVGDGFIRFEFFEDGWDEDEPRRQDLDQYLPQLEKVGKVLIPQMIQKNAEQGRPVS
CLINNPFIPWVSDVAETLGLPSAMLWVQSCACFLAYYHYYHGLVPFPSENAMEIDVQ
LPSMPLLKHDEVPSFLYPTTPYPFLRRAILGQYKNLEKPFCILMDTFQELEHEIIEYTSKI
CPIKTVGPLFKNPKAPNTTVKGDFMKADDCIGWLDSKPASSVVYVSFGSVVYLKQDQ
WDEIAYGLLNSGVNFLWVMKPPHKDSGYTVLTLPEGFLEKAGDRGKVVQWSPQEQV
LAHPATACFVTHCGWNSSMEALTSGMPVVAFPQWGDQVTDAKYLVDEFKVGVRMC
RGEAEDKLITRDVVEQCLREATQGPKAAEMKKNALKWKAAAEASFVEGGSSDRNLQ
AFVDEVKRRSIEITASKPAVKAAPNGVVAAAESVVETKANGKVELAA

SEQ ID NO: 9:

MGSESSLVHVFLVSFPSQGCVNPLLRLGKRLASKGLLVTFTTPESIGKQMRKASNISDQ
PAPVGDGFIRFEFFEDGWDEDEPRRQDLDQYLPQLEKVGKVLIPQMIQKNAEQGRPVS
CLINNPFIPWVSDVAETLGLPSAMLWVQSCACFLAYYHYYHGLVPFPSENAMEIDVQ
LPSMPLLKHDEVPSFLYPTTPYPFLRRAILGQYKNLEKPFCILMDTFQELEHEIIEYTSKI
CPIKTVGPLFKNPKAPNTTVKGDFMKADDCIGWLDSKPASSVVYVSFGSVVYLKQDQ
WDEIAYGLLNSGVNFLWVMKPPHKDSGYTVLTLPEGFLEKAGDRGKVVQWSPQEQV
LAHPATACFVTHCGWNSSMEALTSGMPVVAFPQWGDQVTDAKYLVDEFKVGVRMC
RGEAEDKLITRDVVEQCLREATQGPKAAEMKKNALKWKAAAEASFVEGGSSDRNLQ
AFVDEVKRRSIEITASKPAVKAAPNGVVAAAESVVETKANGKVELAA

SEQ ID NO: 10:

MGSESSLVHVFLVSFPSQGNVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID

VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDSGYTVLTLPEGFLEKAGDRGKVVQWSPQE
QVLAHPATACFVTHCGWNSSMEALTSGMPVVAFPQWGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 11:

MGSESSLVHVFLVSFPSQGIVNPLLRLGKRLASKGLLVTFTTPESIGKQMRKASNISDQ
PAPVGDGFIRFEFFEDGWDEDEPRRQDLDQYLPQLEKVGKVLIPQMIQKNAEQGRPVS
CLINNPFIPWVSDVAETLGLPSAMLWVQSCACFLAYYHYYHGLVPFPSENAMEIDVQ
LPSMPLLKHDEVPSFLYPTTPYPFLRRAILGQYKNLEKPFCILMDTFQELEHEIIEYTSKI
CPIKTVGPLFKNPKAPNTTVKGDFMKADDCIGWLDSKPASSVVYVSFGSVVYLKQDQ
WDEIAYGLLNSGVNFLWVMKPPHKDSGYTVLTLPEGFLEKAGDRGKVVQWSPQEQV
LAHPATACFVTHCGWNSSMEALTSGMPVVAFPQWGDQVTDAKYLVDEFKVGVRMC
RGEAEDKLITRDVVEQCLREATQGPKAAEMKKNALKWKAAAEASFVEGGSSDRNLQ
AFVDEVKRRSIEITASKPAVKAAPNGVVAAAESVVETKANGKVELAA

SEQ ID NO: 12:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDRGYTVLTLPEGFLEKAGDRGKVVQWSPQ
EQVLAHPATACFVTHCGWNSSMEALTSGMPVVAFPQWGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 13:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDSGHTVLTLPEGFLEKAGDRGKVVQWSPQE

QVLAHPATACFVTHCGWNSSMEALTSGMPVVAFPQWGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 14:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDSGRTVLTLPEGFLEKAGDRGKVVQWSPQE
QVLAHPATACFVTHCGWNSSMEALTSGMPVVAFPQWGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 15:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDSGVTVLTLPEGFLEKAGDRGKVVQWSPQE
QVLAHPATACFVTHCGWNSSMEALTSGMPVVAFPQWGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 16:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDHGRTVLTLPEGFLEKAGDRGKVVQWSPQ
EQVLAHPATACFVTHCGWNSSMEALTSGMPVVAFPQWGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 17:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDHGVTVLTLPEGFLEKAGDRGKVVQWSPQ
EQVLAHPATACFVTHCGWNSSMEALTSGMPVVAFPQWGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 18:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDRGHTVLTLPEGFLEKAGDRGKVVQWSPQ
EQVLAHPATACFVTHCGWNSSMEALTSGMPVVAFPQWGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 19:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDRGRTVLTLPEGFLEKAGDRGKVVQWSPQ
EQVLAHPATACFVTHCGWNSSMEALTSGMPVVAFPQWGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 20:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP

VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDRGCTVLTLPEGFLEKAGDRGKVVQWSPQ
EQVLAHPATACFVTHCGWNSSMEALTSGMPVVAFPQWGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 21:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDRGSTVLTLPEGFLEKAGDRGKVVQWSPQE
QVLAHPATACFVTHCGWNSSMEALTSGMPVVAFPQWGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 22:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDRGITVLTLPEGFLEKAGDRGKVVQWSPQE
QVLAHPATACFVTHCGWNSSMEALTSGMPVVAFPQWGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 23:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ

DQWDEIAYGLLNSGVNFLWVMKPPHKDRGVTVLTLPEGFLEKAGDRGKVVQWSPQ
EQVLAHPATACFVTHCGWNSSMEALTSGMPVVAFPQWGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 24:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDNGVTVLTLPEGFLEKAGDRGKVVQWSPQ
EQVLAHPATACFVTHCGWNSSMEALTSGMPVVAFPQWGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 25:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDGGRTVLTLPEGFLEKAGDRGKVVQWSPQ
EQVLAHPATACFVTHCGWNSSMEALTSGMPVVAFPQWGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 26:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDIGRTVLTLPEGFLEKAGDRGKVVQWSPQE
QVLAHPATACFVTHCGWNSSMEALTSGMPVVAFPQWGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 27:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDIGITVLTLPEGFLEKAGDRGKVVQWSPQE
QVLAHPATACFVTHCGWNSSMEALTSGMPVVAFPQWGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 28:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDIGVTVLTLPEGFLEKAGDRGKVVQWSPQE
QVLAHPATACFVTHCGWNSSMEALTSGMPVVAFPQWGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 29:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDLGRTVLTLPEGFLEKAGDRGKVVQWSPQE
QVLAHPATACFVTHCGWNSSMEALTSGMPVVAFPQWGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 30:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDVGRTVLTLPEGFLEKAGDRGKVVQWSPQ
EQVLAHPATACFVTHCGWNSSMEALTSGMPVVAFPQWGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 31:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDVGITVLTLPEGFLEKAGDRGKVVQWSPQE
QVLAHPATACFVTHCGWNSSMEALTSGMPVVAFPQWGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 32:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDVGLTVLTLPEGFLEKAGDRGKVVQWSPQ
EQVLAHPATACFVTHCGWNSSMEALTSGMPVVAFPQWGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 33:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID

VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDVGVTVLTLPEGFLEKAGDRGKVVQWSPQ
EQVLAHPATACFVTHCGWNSSMEALTSGMPVVAFPQWGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 34:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDFGRTVLTLPEGFLEKAGDRGKVVQWSPQE
QVLAHPATACFVTHCGWNSSMEALTSGMPVVAFPQWGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 35:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDFGITVLTLPEGFLEKAGDRGKVVQWSPQE
QVLAHPATACFVTHCGWNSSMEALTSGMPVVAFPQWGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 36:

MGSESSLVHVFLVSFPGQGVVNPLLRLGKRLASKGLLVTFTTPESIGKQMRKASNISD

QPAPVGDGFIRFEFFEDGWDEDEPRRQDLDQYLPQLEKVGKVLIPQMIQKNAEQGRP

VSCLINNPFIPWVSDVAETLGLPSAMLWVQSCACFLAYYHYYHGLVPFPSENAMEID

VQLPSMPLLKHDEVPSFLYPTTPYPFLRRAILGQYKNLEKPFCILMDTFQELEHEIIEYT

SKICPIKTVGPLFKNPKAPNTTVKGDFMKADDCIGWLDSKPASSVVYVSFGSVVYLKQ

DQWDEIAYGLLNSGVNFLWVMKPPHKDFGVTVLTLPEGFLEKAGDRGKVVQWSPQE

QVLAHPATACFVTHCGWNSSMEALTSGMPVVAFPQWGDQVTDAKYLVDEFKVGVR

MCRGEAEDKLITRDVVEQCLREATQGPKAAEMKKNALKWKAAAEASFVEGGSSDR

NLQAFVDEVKRRSIEITASKPAVKAAPNGVVAAAESVVETKANGKVELAA

SEQ ID NO: 37:

MGSESSLVHVFLVSFPGQGVVNPLLRLGKRLASKGLLVTFTTPESIGKQMRKASNISD

QPAPVGDGFIRFEFFEDGWDEDEPRRQDLDQYLPQLEKVGKVLIPQMIQKNAEQGRP

VSCLINNPFIPWVSDVAETLGLPSAMLWVQSCACFLAYYHYYHGLVPFPSENAMEID

VQLPSMPLLKHDEVPSFLYPTTPYPFLRRAILGQYKNLEKPFCILMDTFQELEHEIIEYT

SKICPIKTVGPLFKNPKAPNTTVKGDFMKADDCIGWLDSKPASSVVYVSFGSVVYLKQ

DQWDEIAYGLLNSGVNFLWVMKPPHKDYGITVLTLPEGFLEKAGDRGKVVQWSPQE

QVLAHPATACFVTHCGWNSSMEALTSGMPVVAFPQWGDQVTDAKYLVDEFKVGVR

MCRGEAEDKLITRDVVEQCLREATQGPKAAEMKKNALKWKAAAEASFVEGGSSDR

NLQAFVDEVKRRSIEITASKPAVKAAPNGVVAAAESVVETKANGKVELAA

SEQ ID NO: 38:

MGSESSLVHVFLVSFPGQGVVNPLLRLGKRLASKGLLVTFTTPESIGKQMRKASNISD

QPAPVGDGFIRFEFFEDGWDEDEPRRQDLDQYLPQLEKVGKVLIPQMIQKNAEQGRP

VSCLINNPFIPWVSDVAETLGLPSAMLWVQSCACFLAYYHYYHGLVPFPSENAMEID

VQLPSMPLLKHDEVPSFLYPTTPYPFLRRAILGQYKNLEKPFCILMDTFQELEHEIIEYT

SKICPIKTVGPLFKNPKAPNTTVKGDFMKADDCIGWLDSKPASSVVYVSFGSVVYLKQ

DQWDEIAYGLLNSGVNFLWVMKPPHKDYGVTVLTLPEGFLEKAGDRGKVVQWSPQ

EQVLAHPATACFVTHCGWNSSMEALTSGMPVVAFPQWGDQVTDAKYLVDEFKVGV

RMCRGEAEDKLITRDVVEQCLREATQGPKAAEMKKNALKWKAAAEASFVEGGSSD

RNLQAFVDEVKRRSIEITASKPAVKAAPNGVVAAAESVVETKANGKVELAA

SEQ ID NO: 39:

MGSESSLVHVFLVSFPGQGVVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDSGYTVLTLPEGFLEKAGDRGKVVQWSPQE
QVLAHPATACFVTHCGWNSSMEALTSGMPVVAFPQYGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 40:

MGSESSLVHVFLVSFPSQGNVNPLLRLGKRLASKGLLVTFTTPESIGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDSGYTVLTLPEGFLEKAGDRGKVVQWSPQE
QVLAHPATACFVTHCGWNSSMEALTSGMPVVAFPQYGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 41:

MGSESSLVHVFLVSFPSQGNVNPLLRLGKRLASKGLLVTFTTPESCGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDSGYTVLTLPEGFLEKAGDRGKVVQWSPQE
QVLAHPATACFVTHCGWNSSMEALTSGMPVVAFPQYGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 42:

MGSESSLVHVFLVSFPSQGNVNPLLRLGKRLASKGLLVTFTTPESSGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDSGYTVLTLPEGFLEKAGDRGKVVQWSPQE
QVLAHPATACFVTHCGWNSSMEALTSGMPVVAFPQYGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 43:

MGSESSLVHVFLVSFPSQGNVNPLLRLGKRLASKGLLVTFTTPESGGKQMRKASNISD
QPAPVGDGFIRFEFFEDGWDEDEPRRQDLDQYLPQLEKVGKVLIPQMIQKNAEQGRP

VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDSGYTVLTLPEGFLEKAGDRGKVVQWSPQE
QVLAHPATACFVTHCGWNSSMEALTSGMPVVAFPQYGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 44:

MGSESSLVHVFLVSFPSQGNVNPLLRLGKRLASKGLLVTFTTPESVGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDSGYTVLTLPEGFLEKAGDRGKVVQWSPQE
QVLAHPATACFVTHCGWNSSMEALTSGMPVVAFPQYGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 45:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDSGYTVLTLPEGFLEKAGDRGKVVQWSPQE
QVLAHPATACFVTHCGWNSSMEALTSGMPVVAFPQYGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 46:

MGSESSLVHVFLVSFPSQGNVNPLLRLGKRLASKGLLVTFTTPESYGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ

DQWDEIAYGLLNSGVNFLWVMKPPHKDSGYTVLTLPEGFLEKAGDRGKVVQWSPQE
QVLAHPATACFVTHCGWNSSMEALTSGMPVVAFPQYGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 47:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDRGYTVLTLPEGFLEKAGDRGKVVQWSPQ
EQVLAHPATACFVTHCGWNSSMEALTSGMPVVAFPQYGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 48:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDVGYTVLTLPEGFLEKAGDRGKVVQWSPQ
EQVLAHPATACFVTHCGWNSSMEALTSGMPVVAFPQYGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 49:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDSGRTVLTLPEGFLEKAGDRGKVVQWSPQE
QVLAHPATACFVTHCGWNSSMEALTSGMPVVAFPQYGDQVTDAKYLVDEFKVGVR

MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 50:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDSGVTVLTLPEGFLEKAGDRGKVVQWSPQE
QVLAHPATACFVTHCGWNSSMEALTSGMPVVAFPQYGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 51:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDHGRTVLTLPEGFLEKAGDRGKVVQWSPQ
EQVLAHPATACFVTHCGWNSSMEALTSGMPVVAFPQYGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 52:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDHGVTVLTLPEGFLEKAGDRGKVVQWSPQ
EQVLAHPATACFVTHCGWNSSMEALTSGMPVVAFPQYGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 53:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDRGHTVLTLPEGFLEKAGDRGKVVQWSPQ
EQVLAHPATACFVTHCGWNSSMEALTSGMPVVAFPQYGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 54:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDRGRTVLTLPEGFLEKAGDRGKVVQWSPQ
EQVLAHPATACFVTHCGWNSSMEALTSGMPVVAFPQYGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 55:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDRGDTVLTLPEGFLEKAGDRGKVVQWSPQ
EQVLAHPATACFVTHCGWNSSMEALTSGMPVVAFPQYGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID

SEQ ID NO: 56:

VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDRGCTVLTLPEGFLEKAGDRGKVVQWSPQ
EQVLAHPATACFVTHCGWNSSMEALTSGMPVVAFPQYGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 57:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDRGGTVLTLPEGFLEKAGDRGKVVQWSPQ
EQVLAHPATACFVTHCGWNSSMEALTSGMPVVAFPQYGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 58:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDRGITVLTLPEGFLEKAGDRGKVVQWSPQE
QVLAHPATACFVTHCGWNSSMEALTSGMPVVAFPQYGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 59:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDRGVTVLTLPEGFLEKAGDRGKVVQWSPQ

EQVLAHPATACFVTHCGWNSSMEALTSGMPVVAFPQYGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 60:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDRGFTVLTLPEGFLEKAGDRGKVVQWSPQE
QVLAHPATACFVTHCGWNSSMEALTSGMPVVAFPQYGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 61:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDCGRTVLTLPEGFLEKAGDRGKVVQWSPQ
EQVLAHPATACFVTHCGWNSSMEALTSGMPVVAFPQYGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 62:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDGGRTVLTLPEGFLEKAGDRGKVVQWSPQ
EQVLAHPATACFVTHCGWNSSMEALTSGMPVVAFPQYGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 63:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDIGRTVLTLPEGFLEKAGDRGKVVQWSPQE
QVLAHPATACFVTHCGWNSSMEALTSGMPVVAFPQYGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 64:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDIGVTVLTLPEGFLEKAGDRGKVVQWSPQE
QVLAHPATACFVTHCGWNSSMEALTSGMPVVAFPQYGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 65:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDLGVTVLTLPEGFLEKAGDRGKVVQWSPQ
EQVLAHPATACFVTHCGWNSSMEALTSGMPVVAFPQYGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 66:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDVGRTVLTLPEGFLEKAGDRGKVVQWSPQ
EQVLAHPATACFVTHCGWNSSMEALTSGMPVVAFPQYGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 67:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDVGLTVLTLPEGFLEKAGDRGKVVQWSPQ
EQVLAHPATACFVTHCGWNSSMEALTSGMPVVAFPQYGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 68:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDVGVTVLTLPEGFLEKAGDRGKVVQWSPQ
EQVLAHPATACFVTHCGWNSSMEALTSGMPVVAFPQYGDQVTDAKYLVDEFKVGV
RMCRGEAEDKLITRDVVEQCLREATQGPKAAEMKKNALKWKAAAEASFVEGGSSD
RNLQAFVDEVKRRSIEITASKPAVKAAPNGVVAAAESVVETKANGKVELAA

SEQ ID NO: 69:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ

DQWDEIAYGLLNSGVNFLWVMKPPHKDVGFTVLTLPEGFLEKAGDRGKVVQWSPQE
QVLAHPATACFVTHCGWNSSMEALTSGMPVVAFPQYGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 70:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDFGRTVLTLPEGFLEKAGDRGKVVQWSPQE
QVLAHPATACFVTHCGWNSSMEALTSGMPVVAFPQYGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

SEQ ID NO: 71:

MGSESSLVHVFLVSFPSQGNVNPLLRLGKRLASKGLLVTFTTPESFGKQMRKASNISD
QPAPVGDGFIRFEFFEDGWDEDEPRRQDLDQYLPQLEKVGKVLIPQMIQKNAEQGRP
VSCLINNPFIPWVSDVAETLGLPSAMLWVQSCACFLAYYHYYHGLVPFPSENAMEID
VQLPSMPLLKHDEVPSFLYPTTPYPFLRRAILGQYKNLEKPFCILMDTFQELEHEIIEYT
SKICPIKTVGPLFKNPKAPNTTVKGDFMKADDCIGWLDSKPASSVVYVSFGSVVYLKQ
DQWDEIAYGLLNSGVNFLWVMKPPHKDFGVTVLTLPEGFLEKAGDRGKVVQWSPQE
QVLAHPATACFVTHCGWNSSMEALTSGMPVVAFPQYGDQVTDAKYLVDEFKVGVR
MCRGEAEDKLITRDVVEQCLREATQGPKAAEMKKNALKWKAAAEASFVEGGSSDR
NLQAFVDEVKRRSIEITASKPAVKAAPNGVVAAAESVVETKANGKVELAA

The disclosure of Japanese Patent Application No. 2023-042144 filed on March 16, 2023 is incorporated in the present specification by reference. All documents, patent applications, and technical standards described in the present specification are incorporated herein by reference to the same extent as in a case of being specifically and individually noted that individual documents, patent applications, and technical standards are incorporated herein by reference.

[Sequence list] International application 22F01506W1JP24003369_11.xml based on International Patent Cooperation Treaty

**Claims**

1. A mutant glycosyltransferase having 80% or more sequence identity with an amino acid sequence set forth in SEQ ID NO: 1,
   wherein the mutant glycosyltransferase is capable of forming an anthraquinone C-glycoside from an anthraquinone compound and a monosaccharide donor.

2. The mutant glycosyltransferase according to claim 1,

   wherein at least one requirement selected from the group consisting of the following (1-A) to (6-A) is satisfied,
   (1-A): an amino acid residue corresponding to a 17th position from an N-terminus of SEQ ID NO: 1 is a basic amino acid residue, a polar uncharged amino acid residue, or an aliphatic amino acid residue,
   (2-A): an amino acid residue corresponding to a 20th position from the N-terminus of SEQ ID NO: 1 is a polar uncharged amino acid residue or an aliphatic amino acid residue,
   (3-A): an amino acid residue corresponding to a 318th position from the N-terminus of SEQ ID NO: 1 is a basic amino acid residue, a polar uncharged amino acid residue, an aliphatic amino acid residue, or an aromatic amino acid residue,
   (4-A): an amino acid residue corresponding to a 320th position from the N-terminus of SEQ ID NO: 1 is a basic amino acid residue, an acidic amino acid residue, a polar uncharged amino acid residue, an aliphatic amino acid residue, or an aromatic amino acid residue,
   (5-A): an amino acid residue corresponding to a 382nd position from the N-terminus of SEQ ID NO: 1 is an aromatic amino acid residue,
   (6-A): an amino acid residue corresponding to a 46th position from the N-terminus of SEQ ID NO: 1 is a polar uncharged amino acid residue, an aliphatic amino acid residue, or an aromatic amino acid residue.

3. The mutant glycosyltransferase according to claim 2,

   wherein at least one requirement selected from the group consisting of the following (1-B) to (6-B) is satisfied,
   (1-B): the amino acid residue corresponding to the 17th position from the N-terminus of SEQ ID NO: 1 is a histidine residue (H), a serine residue (S), or a glycine residue (G),
   (2-B): the amino acid residue corresponding to the 20th position from the N-terminus of SEQ ID NO: 1 is a cysteine residue (C), an asparagine residue (N), an isoleucine residue (I), or a valine residue (V),
   (3-B): the amino acid residue corresponding to the 318th position from the N-terminus of SEQ ID NO: 1 is a histidine residue (H), an arginine residue (R), a cysteine residue (C), an asparagine residue (N), a serine residue (S), an isoleucine residue (I), a leucine residue (L), a valine residue (V), a phenylalanine residue (F), or a tyrosine residue (Y),
   (4-B): the amino acid residue corresponding to the 320th position from the N-terminus of SEQ ID NO: 1 is a histidine residue (H), an arginine residue (R), an aspartic acid residue (D), a cysteine residue (C), a serine residue (S), a glycine residue (G), an isoleucine residue (I), a valine residue (V), a phenylalanine residue (F), or a tyrosine residue (Y),
   (5-B): the amino acid residue corresponding to the 382nd position from the N-terminus of SEQ ID NO: 1 is a tyrosine residue (Y),
   (6-B): the amino acid residue corresponding to the 46th position from the N-terminus of SEQ ID NO: 1 is a cysteine residue (C), a serine residue (S), a glycine residue (G), a valine residue (V), a phenylalanine residue (F), or a tyrosine residue (Y).

4. The mutant glycosyltransferase according to claim 3,

   wherein, among the requirements (1-B) to (6-B),
   a combination of only (1-B) and (2-B),
   a combination of only (3-B) and (4-B),
   a combination of only (1-B), (2-B), and (5-B),
   a combination of only (1-B), (2-B), (5-B), and (6-B),

a combination of only (1-B), (2-B), (3-B), (5-B), and (6-B),
a combination of only (1-B), (2-B), (4-B), (5-B), and (6-B), or
a combination of (1-B), (2-B), (3-B), (4-B), (5-B), and (6-B) is satisfied.

5. The mutant glycosyltransferase according to claim 1,
   wherein the mutant glycosyltransferase has one amino acid sequence selected from the group consisting of SEQ ID NO: 1 to SEQ ID NO: 71.

6. A nucleic acid encoding the mutant glycosyltransferase according to any one of claims 1 to 5.

7. A vector comprising:
   the nucleic acid according to claim 6.

8. The vector according to claim 7,
   wherein the vector is an expression vector.

9. A host cell comprising:
   the expression vector according to claim 8.

10. The host cell according to claim 9,
    wherein the host cell is Escherichia coli.

11. A method for producing a mutant glycosyltransferase, comprising:

    culturing the host cell according to claim 9 in a culture medium; and
    recovering the mutant glycosyltransferase from at least one of the cultured host cell or the culture medium.

12. A composition comprising:
    the mutant glycosyltransferase according to any one of claims 1 to 5.

13. A method for producing an anthraquinone C-glycoside, comprising:
    a step of bringing an anthraquinone compound and a monosaccharide donor into contact with the mutant glycosyltransferase according to any one of claims 1 to 5 to form an anthraquinone C-glycoside.

**EP 4 682 259 A1**

## INTERNATIONAL SEARCH REPORT

| International application No. |
|---|
| **PCT/JP2024/003369** |

| A. | CLASSIFICATION OF SUBJECT MATTER |
|---|---|

*C12N 15/54*(2006.01)i; *C12N 1/21*(2006.01)i; *C12N 9/10*(2006.01)i; *C12N 15/63*(2006.01)i; *C12P 19/00*(2006.01)i
FI:  C12N15/54; C12N15/63 Z; C12N1/21; C12P19/00; C12N9/10 ZNA

According to International Patent Classification (IPC) or to both national classification and IPC

| B. | FIELDS SEARCHED |
|---|---|

Minimum documentation searched (classification system followed by classification symbols)

C12N15/00-15/90; C12N9/00-9/99; C12P1/00-41/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII), CAplus/MEDLINE/EMBASE/BIOSIS (STN), UniProt/GeneSeq

| C. | DOCUMENTS CONSIDERED TO BE RELEVANT |
|---|---|

| Category* | Citation of document, with indication, where appropriate, of the relevant passages | Relevant to claim No. |
|---|---|---|
| X | UniProtKB, [online]. Accession No. A 0A 193AUF 6. Definition: 1-beta-glucosyltransferase 84A 23. 22 February 2023, [retrieved on 10 April 2024], Retrieved from the Internet: <URL: https://rest.uniprot.org/unisave/A 0A 193AUF 6?format=txt&versions=37> entire text | 1-3, 6-12 |
| A | | 4-5, 13 |
| X | ONO, N. N. et al. Two UGT 84 Family Glycosyltransferases Catalyze a Critical Reaction of Hydrolyzable Tannin Biosynthesis in Pomegranate (Punica granatum). PLoS One. 2016, vol. 11: e 0156319, pp. 1-25 abstract, p. 4, lines 4-14 | 1-3, 6-12 |
| A | | 4-5, 13 |
| A | YANG, D. et al. Production of Carminic Acid by Metabolically Engineered Escherichia coli. J. Am. Chem. Soc. 2021, vol. 143, pp. 5364-5377 p. 5368, right column, line 12 to p. 5369, right column, line 18, p. 5370, right column, line 24 to p. 5371, left column, line 56, fig. 2-3 | 1-13 |

☑ Further documents are listed in the continuation of Box C.      ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **12 April 2024** | **23 April 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT

| | International application No. |
|---|---|
| | **PCT/JP2024/003369** |

**C. DOCUMENTS CONSIDERED TO BE RELEVANT**

| Category* | Citation of document, with indication, where appropriate, of the relevant passages | Relevant to claim No. |
|---|---|---|
| A | WO 2015/091843 A1 (KOBENHAVNS UNIVERSITET) 25 June 2015 (2015-06-25) claim 1, example 1 | 1-13 |
| A | US 2021/0261992 A1 (MANUS BIO, INC.) 26 August 2021 (2021-08-26) paragraph [0037] | 1-13 |

Form PCT/ISA/210 (second sheet) (July 2022)

**INTERNATIONAL SEARCH REPORT**

International application No.

**PCT/JP2024/003369**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

## INTERNATIONAL SEARCH REPORT
### Information on patent family members

International application No.

**PCT/JP2024/003369**

| Patent document cited in search report | | | Publication date (day/month/year) | Patent family member(s) | | | Publication date (day/month/year) |
|---|---|---|---|---|---|---|---|
| WO | 2015/091843 | A1 | 25 June 2015 | US | 2016/0376569 | A1 | |
| | | | | EP | 3083950 | A1 | |
| | | | | CN | 106062186 | A | |
| US | 2021/0261992 | A1 | 26 August 2021 | WO | 2019/241322 | A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10100290 B
- US 20210261992 A

- JP 2023042144 A


**Non-patent literature cited in the description**

- **DONGSOO YANG** ; **WOO DAE JANG** ; **SANG YUP LEE**. *Journal of American Chemical Society*, 2021, vol. 143 (14), 5364-5377

- **NADIA N. ONO** ; **XIAOQIONG QIN** ; **ALEXANDER E. WILSON** ; **GANG LI1** ; **LI TIAN**. *PLoS ONE*, vol. 11 (5), e0156319